# EUROPEAN PATENT APPLICATION

(11) **EP 2 251 335 A1**
(43) Date of publication of application: **17.11.2010**
(21) Application number: 09709735.6
(22) Date of filing: 07.01.2009
(51) Int. Cl.: C07D 333/16, C07D 333/08, C07D 333/12, C07D 409/14, C07D 471/04, H01L 29/786, H01L 51/05, H01L 51/30, H01L 51/42

(54) **BRANCHED COMPOUNDS, ORGANIC THIN FILMS MADE BY USING THE SAME, AND ORGANIC THIN FILM DEVICES**

(30) Priority: 13.02.2008 JP 2008031973; 29.08.2008 JP 2008221779
(71) Applicant: Osaka University, Suita-shi Osaka 565-0871 (JP); Sumitomo Chemical Company, Limited, Tokyo 104-8260 (JP)
(72) Inventor: IE, Yutaka, Suita-shi Osaka 565-0871 (JP); UTO, Toshihiko, Suita-shi Osaka 565-0871 (JP); ASO, Yoshio, Suita-shi Osaka 565-0871 (JP); UEDA, Masato, Tsukuba-shi Ibaraki 305-0046 (JP)
(74) Representative: Duckworth, Timothy John
(86) International application number: PCT/JP2009/050083
(87) International publication number: WO 2009/101823

(57) **Abstract**

A branched compound including a core part, at least one side chain part bonded to the core part, and an end, wherein one repeating unit or two or more repeating units expressed by the following formula (1) repeat in the or each side chain part, with the proviso that in a repeating unit bonded to the core part, T is bonded to the core part, and in two or more contiguous repeating units, each L is bonded to the T, each L is formed of a plurality of conjugation-forming units linked together; each L includes at least one thienylene unit as the conjugation-forming unit; and at least two of the groups existing at the ends of Ls (the ends of the L in sides which are not bonded to T) are acceptor groups. 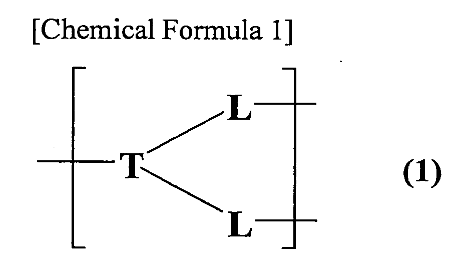 (In the formula, each L represents a divalent organic group which may have a substituent and the T represents a trivalent organic group which may have a substituent.)

## Description

### Technical Field

The present invention relates to a branched compound, organic thin films made by using the same and organic thin film devices.

### Background Art

A thin film containing an organic material having an electric-charge (electron or hole) transportability is expected to be applied to organic thin film devices such as an organic thin film transistor, an organic solar cell and a photosensor, and the development of an organic p-type semiconductor (which shows hole transportability) and an organic n-type semiconductor (which shows electron transportability) is variously studied.

As for the organic p-type semiconductor material, a compound having a thiophene ring such as oligothiophene and polythiophene is expected to show high hole transportability because of stably being in a radical cation state. The oligothiophene having a long chain length particularly has a long conjugation length, and is anticipated to be capable of transporting holes more efficiently.

In recent years, a bipolar organic semiconductor material having both properties of the organic p-type semiconductor and the organic n-type semiconductor has drawn attention, and is variously studied (Non-Patent Document 1).
[Non Patent Document 1]: Yoshihito Kunugi et al., J. Mat. Chem., 2004, vol. 14, p. 2840.

### Disclosure of the Invention

However, high electric-charge transportability is demanded from the viewpoint of the commercialization of an organic thin film device with the use of the bipolar organic semiconductor having the both properties of the organic p-type semiconductor and the organic n-type semiconductor, but it is still hard to say that the above described well-known material has a sufficient performance.

Then, an object of the present invention is to provide a branched compound which can be used as a bipolar organic semiconductor excellent in electric-charge transportability. Another object of the present invention is to provide an organic thin film containing this branched compound, and an organic thin film device provided with this organic thin film.

In order to achieve the above described object, the present invention provides a branched compound including a core part, at least one side chain part bonded to the core part, and an end, wherein one repeating unit or two or more repeating units expressed by the following formula (1) repeat in the or each side chain part, with the proviso that in a repeating unit bonded to the core part, T is bonded to the core part, and in two or more contiguous repeating units, each L is bonded to T; each L is formed of a plurality of conjugation-forming units linked together; each L includes at least one thienylene unit as the conjugation-forming unit; and at least two of the groups existing at the ends of Ls (the end of the L in a side which is not bonded to T) are acceptor groups. 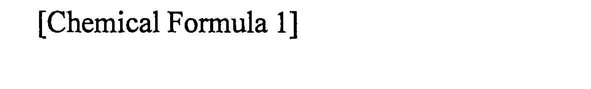 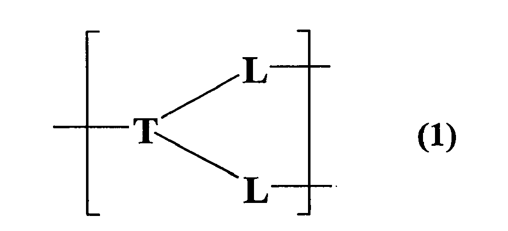 (In the formula, each L represents a divalent organic group which may have a substituent and T represents a trivalent organic group which may have a substituent.)

The branched compound of the present invention has a thiophene ring structure at least in a side chain part, accordingly forms conjugation between rings with adequate planarity, can strengthen an interaction between molecules, and accordingly can be used as an organic p-type semiconductor excellent in electric-charge transportability. In addition, the branched compound contains two or more of L having a group with the acceptor properties at the end, accordingly can have properties (acceptor properties) of the organic n-type semiconductor in the end part in addition to properties (donor properties) of the organic p-type semiconductor in the side chain part, and functions as a bipolar organic semiconductor. The side chain part showing the donor properties is adjacent to the end part showing the acceptor properties, which tends to easily cause an electric-charge transfer between the donor/acceptor, and is expected to show an effect of making the wavelength of an absorption end long, an effect of enhancing the electric-charge separation efficiency of exciton and the like. In addition to these, the branched compound of the present invention is excellent in the stability of the compound and the solubility into an organic solvent, and accordingly, it becomes possible to produce an organic thin film device excellent in performance by forming a thin film with the use of the solution.

In the branched compound of the present invention, it is preferable that one, two or more of the repeating unit which is expressed by the above formula (1) repeat in all the side chain parts.

In the branched compound of the present invention, the core part, T(s) and Ls are preferably in conjugation as a whole to form the branched-type conjugation compound. When the branched compound has such a structure, the planarity enhances as a whole molecule, conjugation properties also enhances, and the branched compound results in being remarkably excellent in electric-charge transportability when having been used as the organic p-type semiconductor.

In the branched compound of the present invention, two or more of the repeating units expressed by the formula (1) are preferably bonded at the T to the core part, and the L is preferably a divalent organic group expressed by the formula (2).

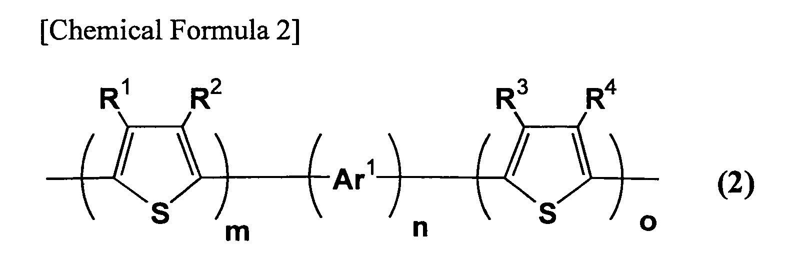

In the formula (2), Ar¹ represents a divalent aromatic hydrocarbon group which may have a substituent, or a divalent heterocyclic group which may have a substituent. R¹, R², R³ and R⁴ each independently represent a hydrogen atom, an alkyl group, an alkoxy group, an aryl group which may have a substituent, or a monovalent heterocyclic group which may have a substituent, and part or all of hydrogen atoms in each of these groups may be substituted with a fluorine atom. Incidentally, when there are a plurality of R¹s, a plurality of R²s, a plurality of R³s and a plurality of R⁴s, the R¹s may be the same or different from each other, the R²s may be the same or different from each other, the R³s may be the same or different from each other, and the R⁴s may be the same or different from each other. In addition, m, n and o each independently represent an integer of 0 to 10, with the proviso that at least one of m and o is an integer of 1 or more, and m + n + o represents an integer of 2 to 16.

The branched compound having such a structure has adequate conjugation properties, and particularly is excellent in the stability of the compound as well. Accordingly, the branched compound is further excellent in electric-charge transportability, and shows excellent characteristics when having been applied to the organic p-type semiconductor.

In the branched compound of the present invention, T is preferably any one of trivalent organic groups, which is expressed by the following formulae (3) to (7), and is particularly preferably a trivalent organic group which is expressed by the following formula (4'). 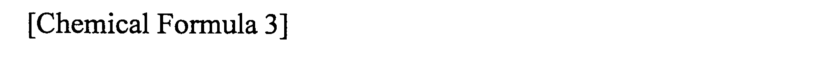 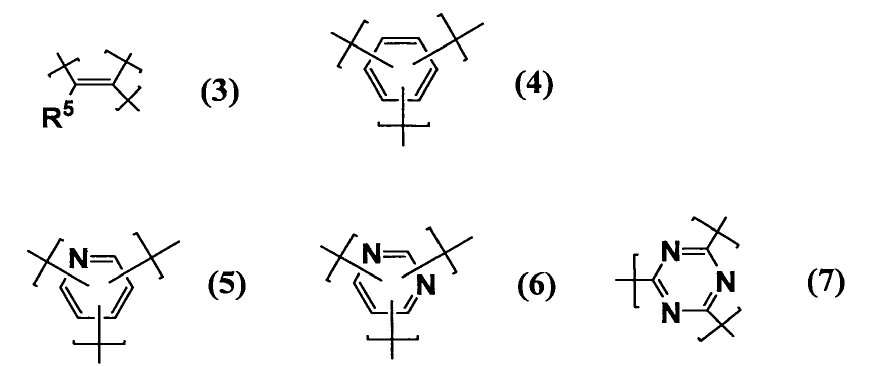 In the formula (3), R⁵ represents a hydrogen atom, an alkyl group, an aryl group or a cyano group.

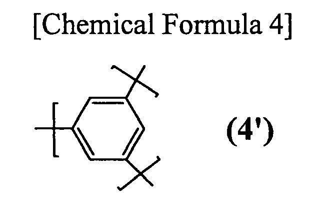

In addition, the divalent organic group which is expressed by the following formula (8) is particularly preferable as a core part.

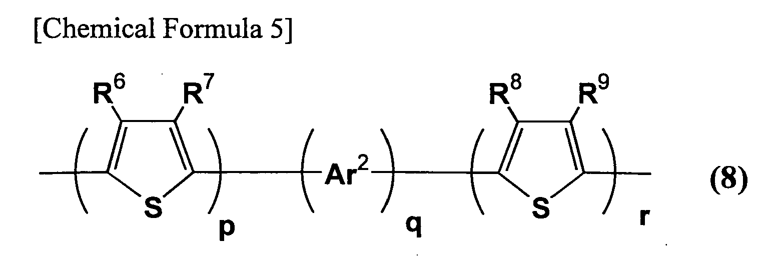

In the formula (8), Ar² represents a divalent aromatic hydrocarbon group which may have a substituent, or a divalent heterocyclic group which may have a substituent. R⁶, R⁷, R⁸ and R⁹ each independently represent a hydrogen atom, an alkyl group, an alkoxy group, an aryl group which may have a substituent, or a monovalent heterocyclic group which may have a substituent, and part or all of hydrogen atoms in each of these groups may be substituted with a fluorine atom. When there are a plurality of R⁶s, a plurality of R⁷s, a plurality of R⁸s and a plurality of R⁹s, the R⁶s may be the same or different from each other, the R⁷s may be the same or different from each other, the R⁸s may be the same or different from each other, and the R⁹s may be the same or different from each other. In addition, p, q and r each independently represent an integer of 0 to 10, with the proviso that p + q + r represents an integer of 2 to 20.

The branched compound including the above described core part becomes more excellent in conjugation properties, and can be used as an organic p-type semiconductor further more excellent in electric-charge transportability. When L is a divalent organic group which is expressed by the formula (2) in particular, the branched compound becomes excellent in uniformity as the whole molecule, the conjugation properties of the whole molecule enhance, and electric-charge transportability largely enhances when the branched compound has been used as the organic p-type semiconductor.

The repeating unit which is expressed by the above formula (1) is preferably a repeating unit which is expressed by the formula (9).

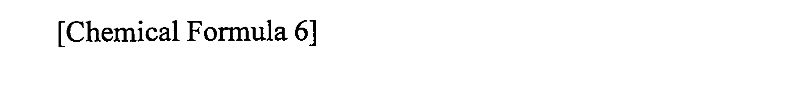

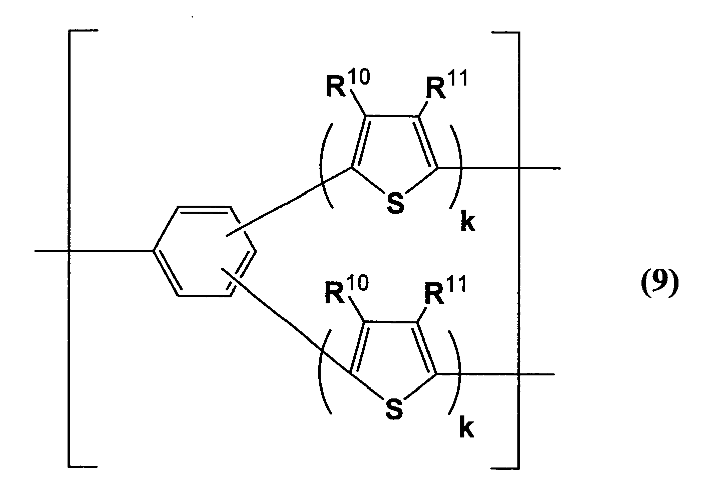

In the formula (9), R¹⁰ and R¹¹ each independently represent a hydrogen atom, an alkyl group or an aryl group, and part or all of hydrogen atoms in each of these groups may be substituted with a fluorine atom. R¹⁰ and R¹¹ which plurally exist may be each the same or different. In addition, k represents an integer of 3 to 10. A plurality of k may be the same or different.

At least two groups existing at the ends of Ls may be an acceptor group, and are preferably a group containing a derivative residue of fullerenes such as C60 and C70, a group containing a naphthalene imide derivative residue or a group containing a perylene imide derivative residue. When the compound has two or more of L having the acceptor group at the end, the acceptor groups become easy to mutually interact with each other between molecules, and thereby the compound can have properties of the organic n-type semiconductor in the end part in addition to properties of the organic p-type semiconductor in the core part and the side chain part, and functions as a bipolar organic semiconductor. A group other than the acceptor group existing in the end of L is preferably a phenyl group. In order to effectively develop properties of the organic p-type semiconductor in the side chain part and properties of the organic n-type semiconductor in the end part, conjugation between the side chain part and the end part is preferably broken.

The present invention further provides an organic thin film device, an organic thin film transistor, an organic solar cell and a photosensor, which have the above described organic thin film.

Such an organic thin film, an organic thin film transistor, an organic solar cell and a photosensor are formed by using the branched compound of the present invention showing excellent bipolar electric-charge transportability as was described above, and accordingly can acquire excellent performance.

### Effect of the Invention

The present invention can provide a new branched compound which can be used as a bipolar organic semiconductor excellent in electric-charge transportability. In addition, the present invention can provide an organic thin film containing this branched compound, and an organic thin film device having this organic thin film. In addition, this organic thin film device can be excellent in stability.

### Brief Description of the Drawings

Figure 1 is a schematic sectional view of an organic thin film transistor according to a first embodiment;
Figure 2 is a schematic sectional view of an organic thin film transistor according to a second embodiment;
Figure 3 is a schematic sectional view of an organic thin film transistor according to a third embodiment;
Figure 4 is a schematic sectional view of an organic thin film transistor according to a fourth embodiment;
Figure 5 is a schematic sectional view of an organic thin film transistor according to a fifth embodiment;
Figure 6 is a schematic sectional view of an organic thin film transistor according to a sixth embodiment;
Figure 7 is a schematic sectional view of an organic thin film transistor according to a seventh embodiment;
Figure 8 is a schematic sectional view of a solar cell according to an embodiment;
Figure 9 is a schematic sectional view of a photosensor according to a first embodiment;
Figure 10 is a schematic sectional view of a photosensor according to a second embodiment;
Figure 11 is a schematic sectional view of a photosensor according to a third embodiment;
Figure 12 is a view illustrating spectral responsivity characteristics of an organic thin film device 2; and
Figure 13 is a view illustrating spectral responsivity characteristics of organic thin film devices 1, 3 and 4.

### Description of Symbols

1 ... substrate, 2 ... active layer, 2a ... active layer, 3 ... insulation layer, 4 ... gate electrode, 5 ... source electrode, 6 ... drain electrode, 7a ... first electrode, 7b ... second electrode, 8 ... electric-charge-generating layer, 100 ... organic thin film transistor according to first embodiment, 110 ... organic thin film transistor according to second embodiment, 120 ... organic thin film transistor according to third embodiment, 130 ... organic thin film transistor according to fourth embodiment, 140 ... organic thin film transistor according to fifth embodiment, 150 ... organic thin film transistor according to sixth embodiment, 160 ... organic thin film transistor according to seventh embodiment, 200 ... solar cell according to embodiment, 300 ... photosensor according to first embodiment, 310 ... photosensor according to second embodiment, and 320 ... photosensor according to third embodiment

### Best Modes for Carrying Out the Invention

Preferred embodiments according to the present invention will be described in detail below occasionally with reference to the drawings. Incidentally, in the drawings, the same reference numerals shall be put on the same elements, and overlapping descriptions are omitted. In addition, a positional relation such as up, down, left and right shall be based on the positional relation as is illustrated in the drawings, unless otherwise specifically indicated. Furthermore, a dimensional ratio in the drawings is not limited to the ratio shown in the drawings.

The branched compound of the present invention includes a core part, at least one side chain part bonded to the core part and an end, and is a compound which can employ a structure of a so-called dendrimer (dendric polymer), hyper-brunch polymer or starburst polymer. The skeleton of the side chain part is expressed by the formula (1), and a preferred side chain part is as described above. The core part is preferably an x-valent organic group (where x is an integer of 1 or more and corresponds to the number of side chain parts, and hereinafter the same). Examples of the core part include an x-valent aromatic hydrocarbon group, an x-valent heterocyclic group, an x-valent arylamine or a residue of the derivative thereof, and an organic group having combined them. A group existing at the end of L is not particularly limited as long as at least two groups thereof are acceptor groups, and are preferably a group containing a derivative residue of fullerenes such as C60 and C70, a group containing a naphthalene imide derivative residue or a group containing a perylene imide derivative residue.

The x-valent aromatic hydrocarbon group means an atom group which is left after x pieces of hydrogen atoms have been removed from a benzene ring or a fused ring, ordinarily has 6 to 60 carbon atoms, and preferably has 6 to 20 carbon atoms. The fused ring includes, for instance, a naphthalene ring, an anthracene ring, a tetracene ring, a pentacene ring, a pyrene ring, a perylene ring, a rubrene ring and a fluorene ring. Among these, an atom group which is left after x pieces of hydrogen atoms have been removed from the benzene ring is particularly preferable. For information, the x-valent aromatic hydrocarbon group may have a substituent thereon. Here, the number of carbon atoms in the substituent is not included in the number of carbon atoms in the aromatic hydrocarbon group with x or more valencies. In addition, the substituent includes a halogen atom, a saturated or unsaturated hydrocarbon group, an aryl group, an alkoxy group, an aryloxy group, a monovalent heterocyclic group, an amino group, a nitro group and a cyano group.

In addition, the x-valent heterocyclic group means an atom group which is left after x pieces of hydrogen atoms have been removed from a heterocyclic compound, ordinarily has 3 to 60 carbon atoms, and preferably has 3 to 20 carbon atoms. The heterocyclic compound includes, for instance, thiophene, thienothiophene, dithienothiophene, pyrrole, pyridine, pyrimidine, pyrazine, triazine, benzothiazole and benzothiadiazole. Among these, an atom group which is left after x pieces of hydrogen atoms have been removed from thiophene, pyridine, pyrimidine and triazine is particularly preferable. In addition, the x-valent heterocyclic group may have a substituent thereon, and the number of carbon atoms in the substituent is not included in the number of carbon atoms in the x-valent heterocyclic group. For information, the substituent includes a halogen atom, a saturated or unsaturated hydrocarbon group, an aryl group, an alkoxy group, an aryloxy group, a monovalent heterocyclic group, an amino group, a nitro group and a cyano group.

The x-valent arylamine or a group formed of a derivative thereof means an atom group which is left after x pieces of hydrogen atoms have been removed from a compound in which amine has been substituted with one or more aryl groups, or a derivative such as a compound in which a plurality of the compounds are bonded to each other. Examples of the arylamine or the derivative thereof include diphenylamine, triphenylamine, N,N'-tetraphenyl-phenylenediamine and N,N'-tetraphenyl-biphenylene diamine, and triphenylamine is preferable.

When the core part is a unit expressed by the following formula (8), conjugation properties of the branched compound further enhances, and electric-charge transportability enhances, which is more preferable.

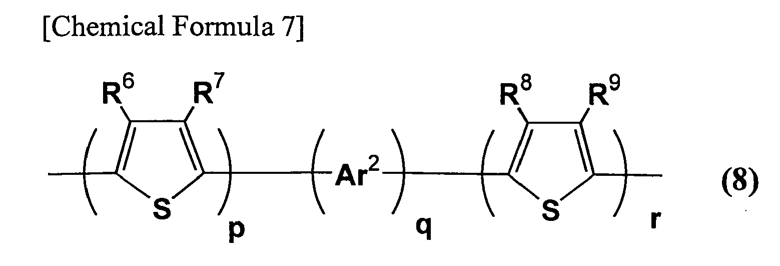

In the formula (8), Ar² represents a divalent aromatic hydrocarbon group which may have a substituent or a divalent heterocyclic group which may have a substituent. R⁶, R⁷, R⁸ and R⁹ each independently represent a hydrogen atom, an alkyl group, an alkoxy group, an aryl group which may have a substituent, or a monovalent heterocyclic group which may have a substituent, and part or all of hydrogen atoms in each of these groups may be substituted with a fluorine atom. When there are a plurality of R⁶s, a plurality of R⁷s, a plurality of R⁸s and a plurality of R⁹s, the R⁶s may be the same or different from each other, the R⁷s may be the same or different from each other, the R⁸s may be the same or different from each other, and the R⁹s may be the same or different from each other. In addition, p, q and r each independently represent an integer of 0 to 10, with proviso that p + q + r is an integer of 2 to 20.

From the viewpoint of enhancing electric-charge transportability, the core part is preferably the case in which q is 0 in the above formula (8), in other words, is preferably all formed of thiophene rings, and it is further preferable that p + q + r is an integer of 2 to 16. From the viewpoint of enhancing solubility into an organic solvent, it is preferable that at least one of R⁶, R⁷, R⁸ and R⁹ is not a hydrogen atom.

In the above formulae (2) and (8), the divalent aromatic hydrocarbon group expressed by Ar¹ and Ar² means an atom group which is left after two pieces of hydrogen atoms have been removed from a benzene ring or a fused ring, usually has 6 to 60 carbon atoms, and preferably has 6 to 20 carbon atoms. The fused ring includes, for instance, a naphthalene ring, an anthracene ring, a tetracene ring, a pentacene ring, a pyrene ring, a perylene ring, a rubrene ring and a fluorene ring. The divalent aromatic hydrocarbon group is preferably the atom group which is left after two pieces of hydrogen atoms have been removed from the benzene ring or the fluorene ring. The divalent aromatic hydrocarbon group may have a substituent thereon. Here, the number of carbon atoms in the substituent is not included in the number of carbon atoms in the divalent aromatic hydrocarbon group. For information, the substituent includes a halogen atom, a saturated or unsaturated hydrocarbon group, an aryl group, an alkoxy group, an aryloxy group, a monovalent heterocyclic group, an amino group, a nitro group and a cyano group.

In addition, the divalent heterocyclic group which is expressed by Ar¹ and Ar² means an atom group which is left after two pieces of hydrogen atoms have removed from the heterocyclic compound, ordinarily has 3 to 60 carbon atoms, and preferably has 3 to 20 carbon atoms. The heterocyclic compound includes, for instance, thiophene, thienothiophene, dithienothiophene, pyrrole, pyridine, pyrimidine, pyrazine, triazine, benzothiazole and benzothiadiazole. The divalent heterocyclic group is preferably an atom group which is left after two pieces of hydrogen atoms have been removed from thiophene or thienothiophene. The divalent heterocyclic group may have a substituent thereon, and the number of carbon atoms in the substituent is not included in the number of carbon atoms in the divalent heterocyclic group. For information, the substituent includes a halogen atom, a saturated or unsaturated hydrocarbon group, an aryl group, an alkoxy group, an aryloxy group, a monovalent heterocyclic group, an amino group, a nitro group and a cyano group.

The alkyl group represented by R¹ to R¹¹ is preferably a straight-chain, branched or cyclic alkyl group having 1 to 20 carbon atoms; includes, for instance, a methyl group, an ethyl group, an n-propyl group, an iso-propyl group, an n-butyl group, an iso-butyl group, a tert-butyl group, a sec-butyl group, a pentyl group, a hexyl group, a heptyl group, an octyl group, a nonyl group, a decyl group, a lauryl group, a cyclopropyl group, a cyclobutyl group, a cyclopentyl group, a cyclohexyl group, a cycloheptyl group, a cyclooctyl group, a cyclononyl group and a cyclododecyl group; is more preferably an alkyl group having 1 to 12 carbon atoms; and is further preferably the pentyl group, the hexyl group, the octyl group, the decyl group and the cyclohexyl group.

Examples of the alkoxy group represented by R¹ to R⁴ and R⁶ to R⁹ include an alkoxy group containing the above described alkyl group in its structure.

The aryl group represented by R¹ to R¹¹ is preferably an aryl group having 6 to 60 carbon atoms, of which examples include, for instance, a phenyl group, a C¹ to C¹² alkoxyphenyl group (C¹ to C¹² means that the number of carbon atoms is 1 to 12. Hereinafter the same.), a C¹ to C¹² alkyl phenyl group, a 1-naphthyl group and a 2-naphthyl group; is preferably an aryl group having 6 to 20 carbon atoms; is more preferably the phenyl group, the C¹ to C¹² alkoxyphenyl group, the C¹ to C¹² alkyl phenyl group; and is further preferably the phenyl group.

The monovalent heterocyclic group represented by R¹ to R⁴ and R⁶ to R⁹ is preferably a monovalent heterocyclic group having 4 to 60 carbon atoms, of which examples include a thienyl group, a C¹ to C¹² alkyl thienyl group, a pyrrolyl group, a furyl group, a pyridyl group, a C¹ to C¹² alkyl pyridyl group; is preferably a monovalent heterocyclic group having 4 to 20 carbon atoms; and is more preferably the thienyl group, the C¹ to C¹² alkyl thienyl group, the pyridyl group and the C¹ to C¹² alkyl pyridyl group.

In addition, in the branched compound of the present invention, at least two groups existing at the ends of Ls may be an acceptor group of which examples include, for instance, an oxadiazole derivative, an anthraquinodimethane derivative, a benzoquinone derivative, a naphthoquinone derivative, an anthraquinone derivative, a tetracyanoanthraquinodimethane derivative, a fluorenone derivative, a diphenyl dicyanoethylene derivative, a diphenoquinone derivative, a 8-hydroxyquinoline derivative, a derivative of fullerenes of C60, C70 and the like, a group containing a naphthalene imide derivative residue and a group containing a perylene imide derivative residue; is preferably the group containing the fullerenes derivative residue of C60, C70 and the like, the group containing the naphthalene imide derivative residue, the group containing the perylene imide derivative residue; and is more preferably a group containing a perylene pigment derivative residue. From the viewpoint of easy synthesis, the group containing the naphthalene imide derivative residue is particularly preferable. From the viewpoint of enhancing the electron-withdrawing properties of the acceptor group, one part or all of hydrogen atoms in the acceptor group may be substituted with fluorine atoms.

In the branched compound of the present invention, a group other than the acceptor group existing at the end of L includes a hydrogen atom or a monovalent organic group. An alkyl group, an alkoxy group, a phenyl group or a substituted phenyl group is preferable as the monovalent organic group. The substituent includes a halogen atom, a saturated or unsaturated hydrocarbon group, an aryl group, an alkoxy group, an aryloxy group, a monovalent heterocyclic group, an amino group, a nitro group and a cyano group, and part or all of hydrogen atoms in each of these groups may be substituted with a fluorine atom. From the viewpoint of the stability of the branched compound, the monovalent organic group is more preferably the phenyl group or the substituted phenyl group, and is further preferably the phenyl group.

Examples of the group containing the fullerenes derivative residue of C60, C70 and the like include the following formulae. 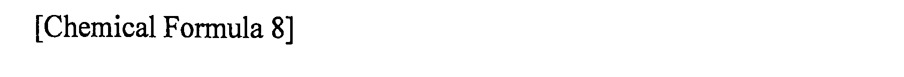 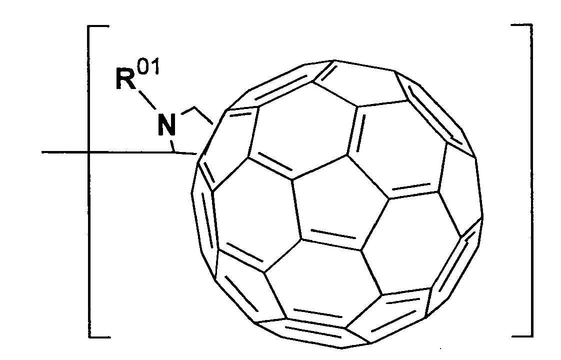 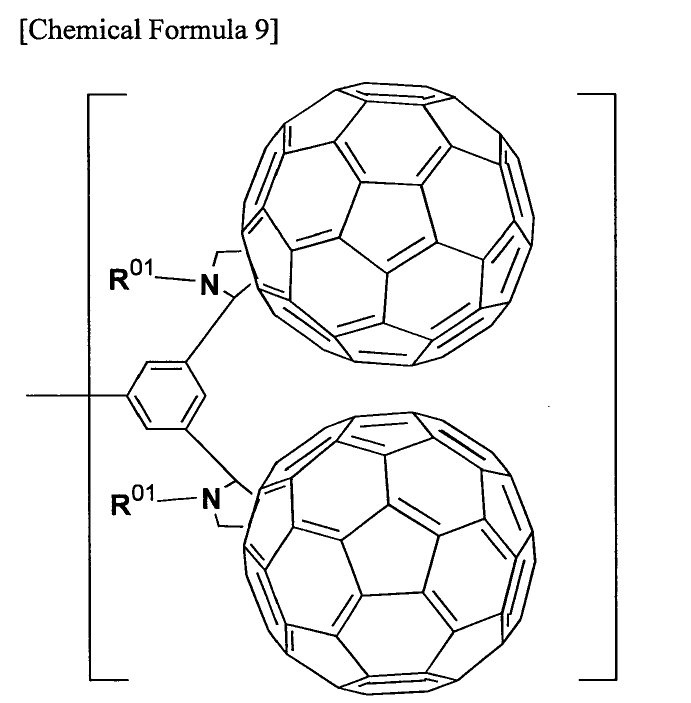 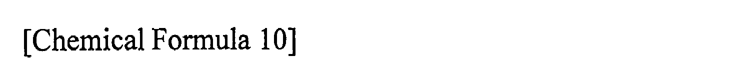 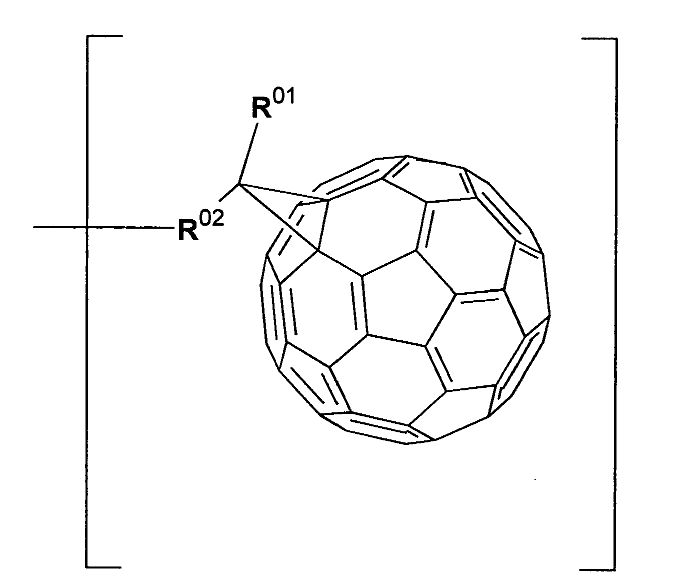 Here, R⁰¹ represents a monovalent organic group, and R⁰² represents a divalent organic group.

Examples of the group containing the perylene imide derivative residue include the following formulae.

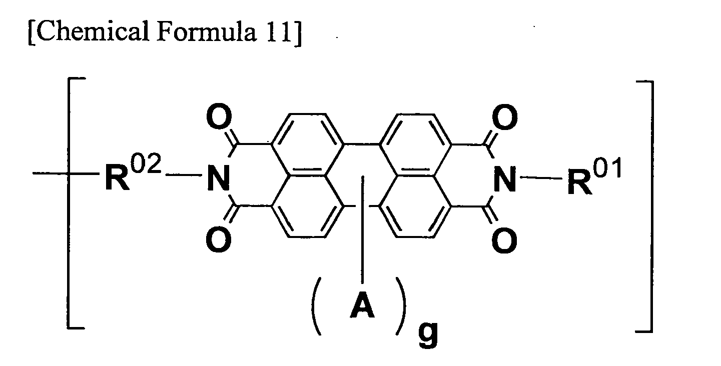

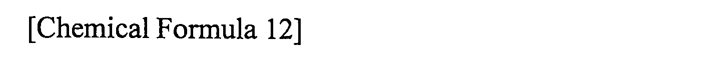

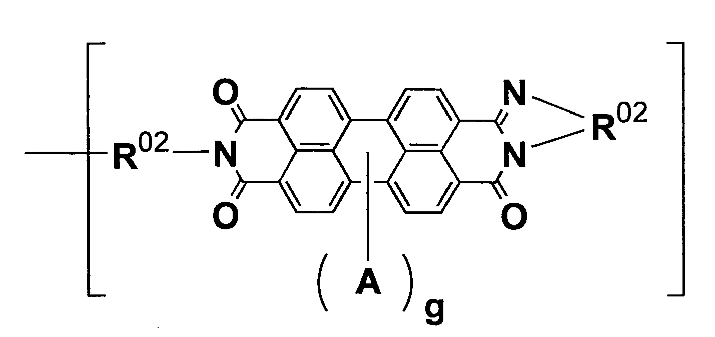

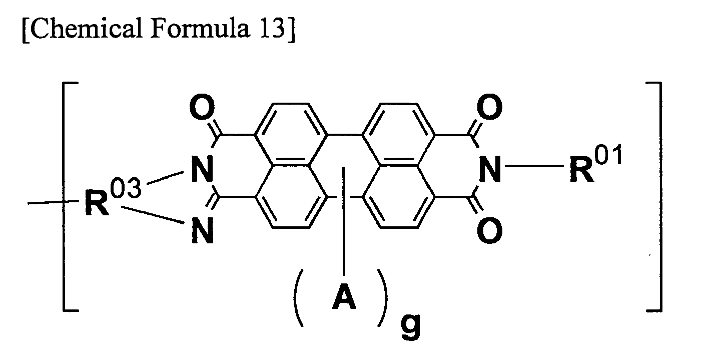

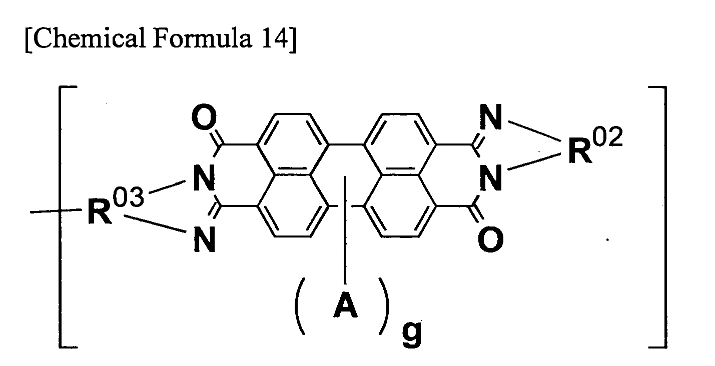

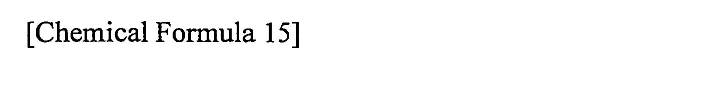

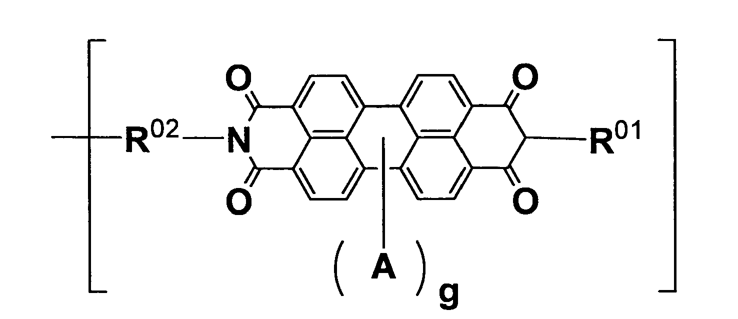

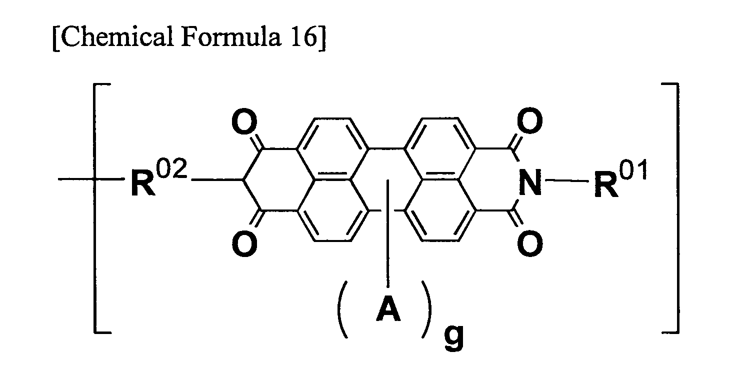

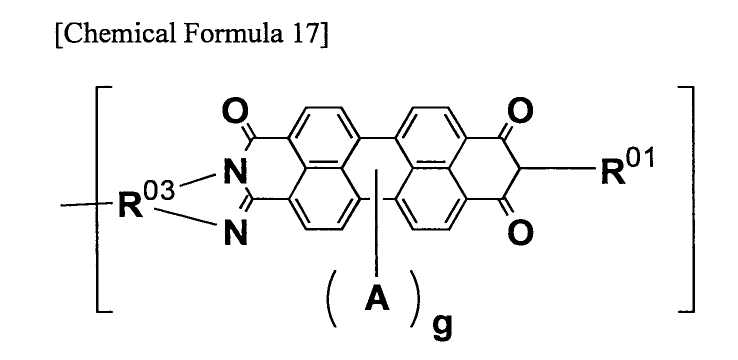

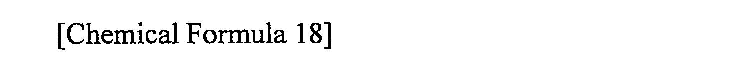

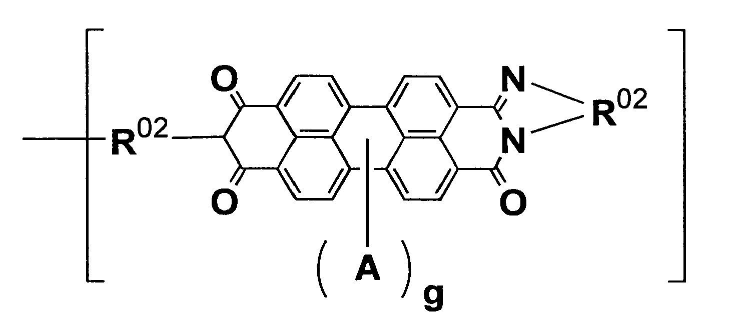

Here, R⁰¹ represents a monovalent organic group, R⁰² represents a divalent organic group, and R⁰³ represents a trivalent organic group; and when there are a plurality of organic groups, they may be each the same or different. A represents an alkyl group, an alkoxy group, a sulfonyl group, an amino group, an ammonium group, a hydroxy group, a nitro group or halogen, and g represents an integer of 0 to 8.

Examples of the group containing the naphthalene imide derivative residue include the following formulae.

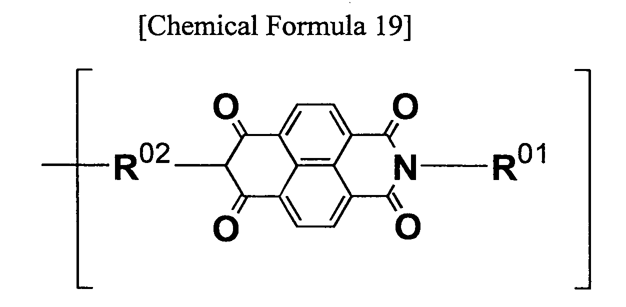

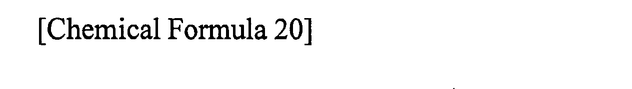

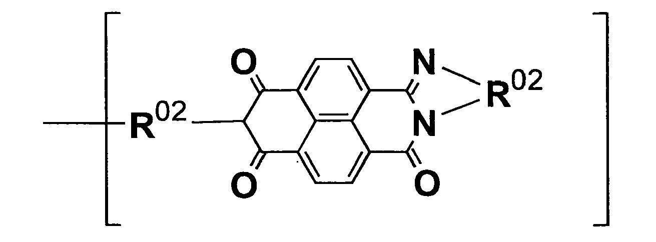

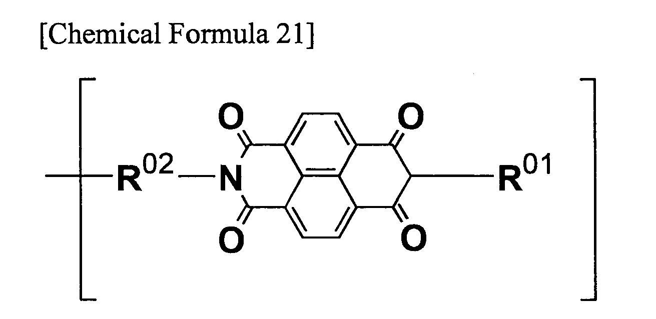

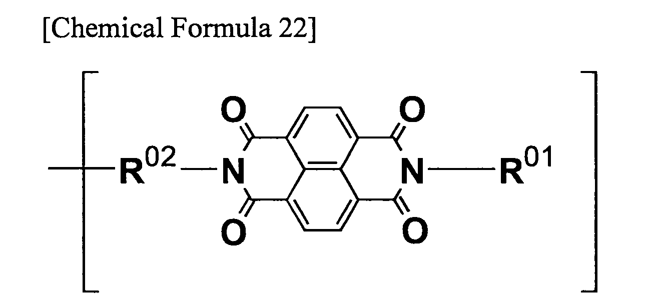

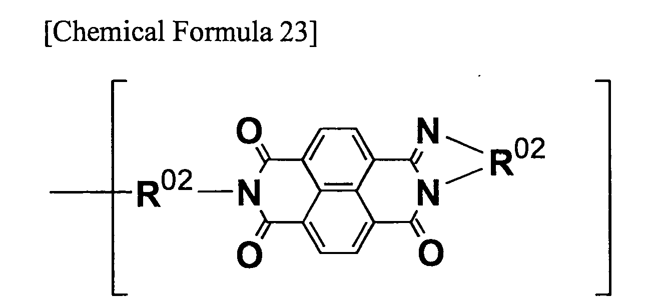

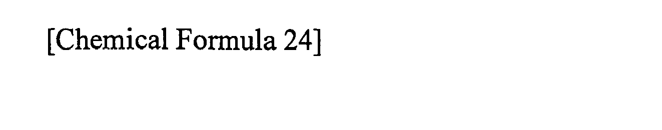

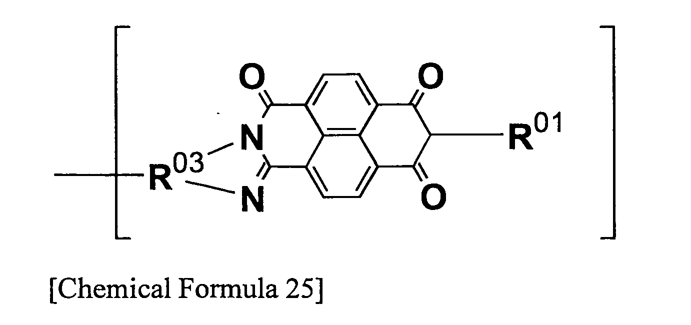

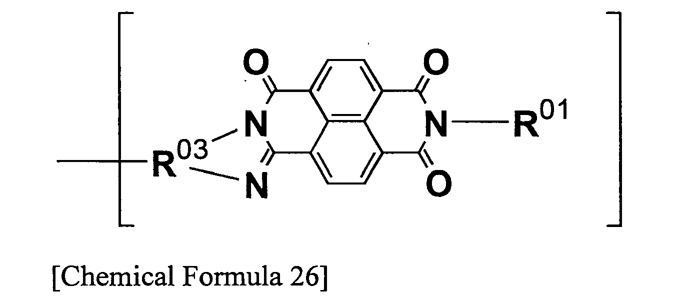

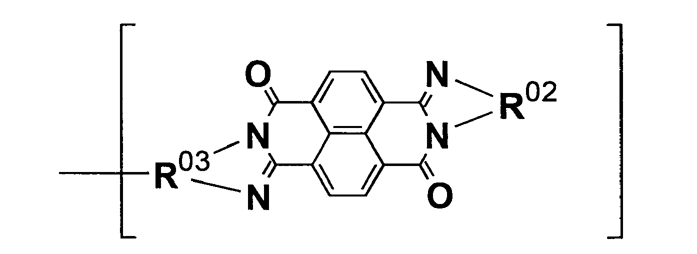

Here, R⁰¹ represents a monovalent organic group, R⁰² represents a divalent organic group, and R⁰³ represents a trivalent organic group; and when there are a plurality of organic groups, they may be each the same or different.

Examples of the monovalent organic group represented by R⁰¹ include a straight-chain, branched or cyclic alkyl group, an alkoxy group, an aryl group, a monovalent heterocyclic group, an amino group, a nitro group and a cyano group; and part or all of hydrogen atoms in each of these groups may be substituted with a fluorine atom. The aryl group and the monovalent heterocyclic group may have a substituent. From the viewpoint of enhancing the electron-withdrawing properties, one part or all of hydrogen atoms in the alkyl group, the alkoxy group and the aryl group are preferably substituted with fluorine atoms, and one part or all of hydrogen atoms in the alkyl group are more preferably substituted with fluorine atoms.

Examples of the divalent organic group represented by R⁰² include an alkylene group, a vinylene group, an ether group, a sulfide group, a carbonyl group, a thiocarbonyl group, a sulfenyl group, a sulfonyl group, a monosubstituted amino group, and an atom group which is left after two pieces of hydrogen atoms have been removed from a ring structure such as a benzene ring, a fused ring or a heterocyclic ring, and one part or all of hydrogen atoms in these atom groups may be substituted with fluorine atoms. Among these, the alkylene group and the atom group which is left after two pieces of hydrogen atoms have been removed from the benzene ring are particularly preferable. In addition, the group having the ring structure may have a substituent thereon. The substituent includes a halogen atom, a saturated or unsaturated hydrocarbon group, an aryl group, an alkoxy group, an aryloxy group, a monovalent heterocyclic group, an amino group, a nitro group and a cyano group.

Examples of the trivalent organic group represented by R⁰³ include an atom group which is left after three pieces of hydrogen atoms have been removed from a ring structure of a benzene ring, a fused ring, a heterocyclic ring or the like, and one part or all of hydrogen atoms in these atom groups may be substituted with fluorine atoms. Among these, an atom group which is left after three pieces of hydrogen atoms have been removed from the benzene ring is particularly preferable. In addition, the group having the ring structure may have a substituent thereon. The substituent includes a halogen atom, a saturated or unsaturated hydrocarbon group, an aryl group, an alkoxy group, an aryloxy group, a monovalent heterocyclic group, an amino group, a nitro group and a cyano group.

Next, the structure of the branched compound of the present invention will be described further in detail. The branched compound of the present invention contains a repeating unit expressed by the above formula (1), as described above, may have at least two Ls having an acceptor group at the end, and may contain two or more types of repeating units expressed by the above formula (1). In addition, at least two groups existing at the ends of a plurality of Ls may be acceptor groups, and may be the same or different. The branched compound of the present invention preferably has four or more Ls having the acceptor groups at the end from the viewpoint of enhancing electron transportability, and it is more preferable that all Ls have the acceptor groups. A plurality of end groups are preferably the same from the viewpoint of the ease of production and the ease of an interaction between molecules.

Examples of the branched compound of the present invention include branched compounds expressed by the following formulae (a), (b), (c) and (d).

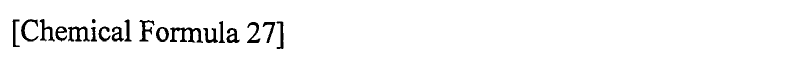

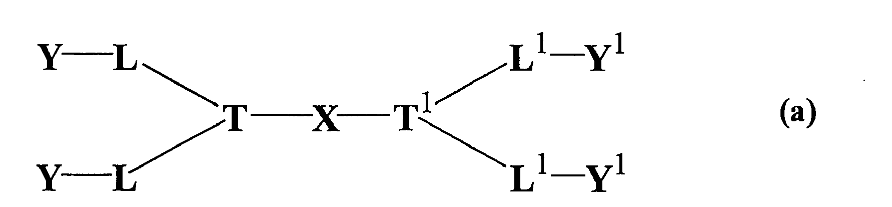

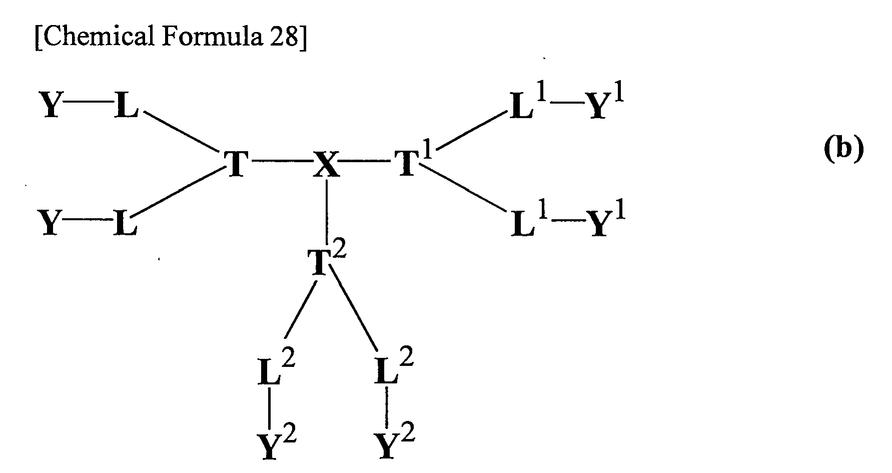

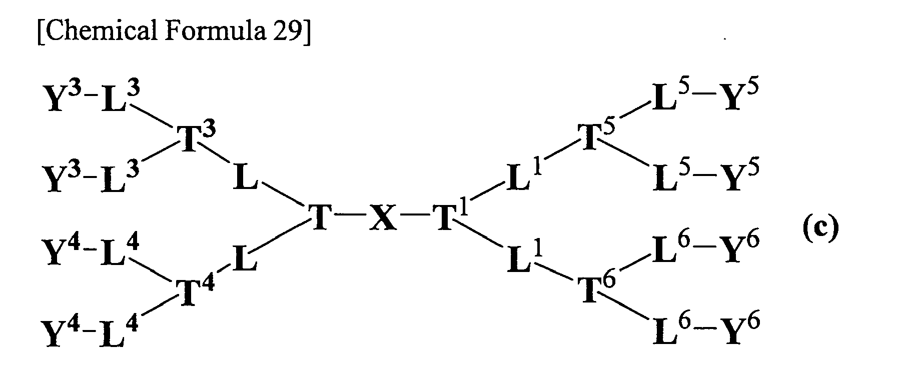

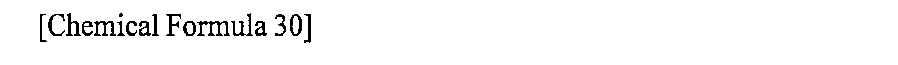

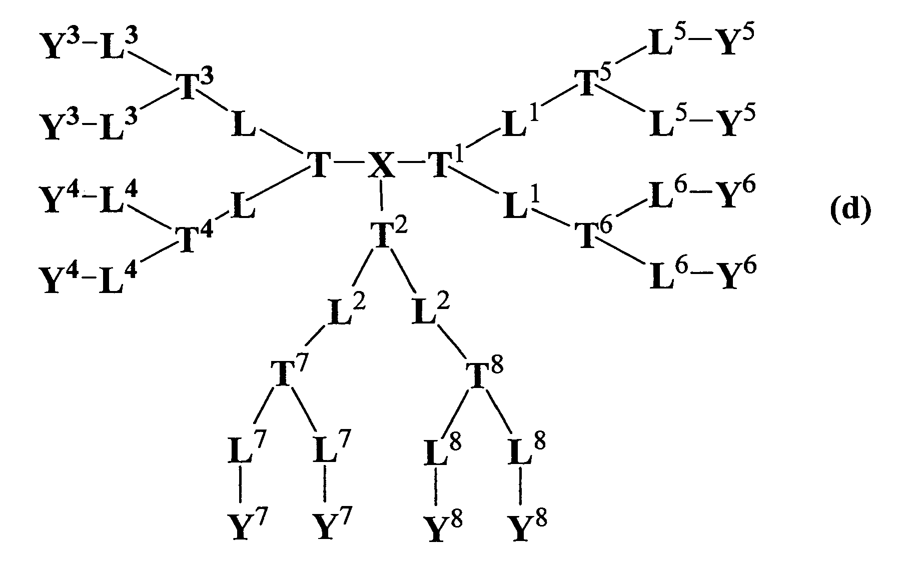

Here, X represents a core part, and Y represents a group existing at the ends of Ls. T^{X}, L^{X} and Y^{X} (X is an integer of 1 to 8) are synonymous with T, L and Y respectively, and may be the same as or different from T, L and Y. From the viewpoint of the ease of production, T^{X}, L^{X} and Y^{X} are preferably the same as T, L and Y, respectively.

The branched compound of the present invention particularly preferably is a compound expressed by the following formula (e) or (f) from the viewpoint of enhancing electric-charge transportability and being excellent in stability.

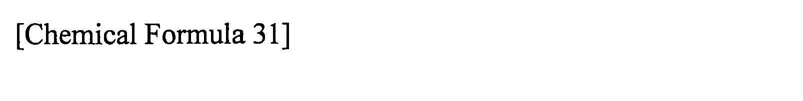

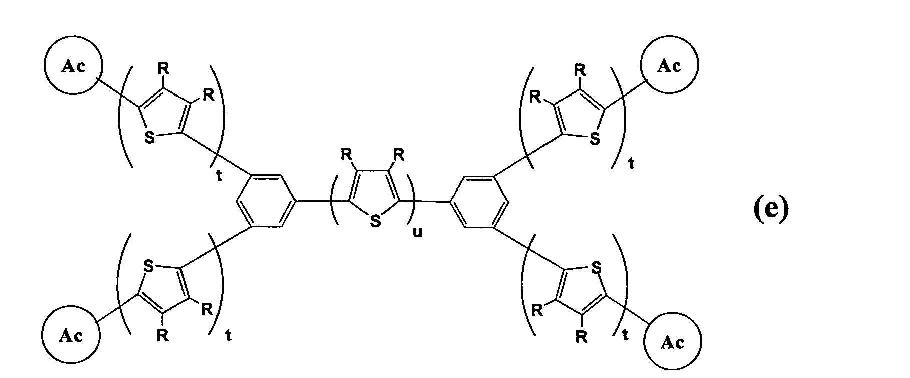

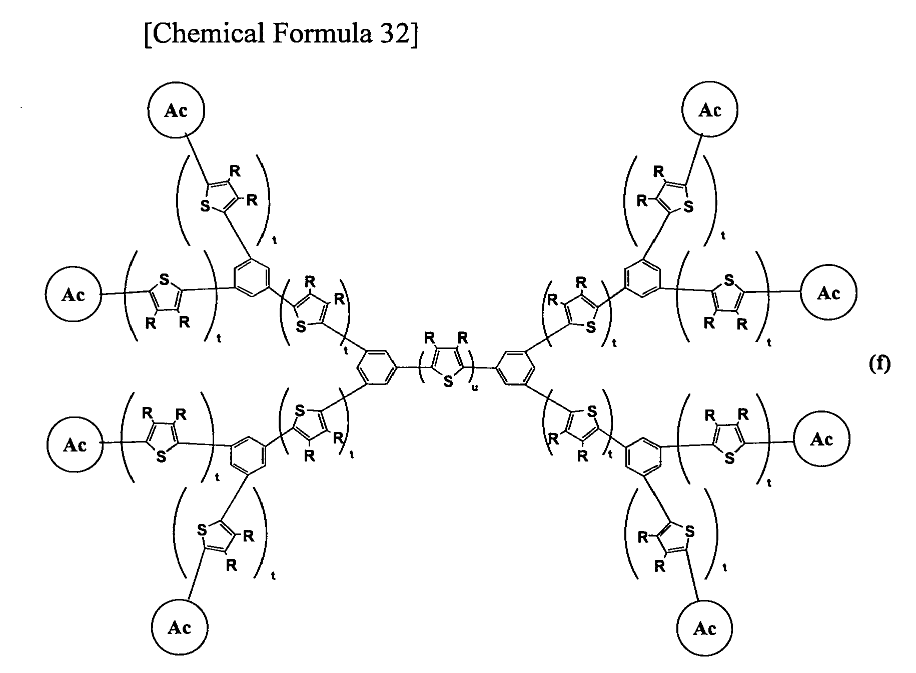

In the formulae (e) and (f), R represents a hydrogen atom or an alkyl group, and a plurality of Rs may be the same or different. At least one of substituents Rs in a series of a plurality of thiophene rings which are bonded is preferably not a hydrogen atom. Ac represents an acceptor group or a phenyl group, and a plurality of Acs may be the same or different, with the proviso that at least two Acs are acceptor groups. t and u each independently represent an integer of 2 to 16.

As for a method for producing the branched compound of the present invention, the branched compound is preferably produced with a production method which will be described below.

The branched compound of the present invention can be produced by schemes A, A', A", B or B' which will be shown below, while employing a compound containing a repeating unit expressed by the above formula (1) as a monomer. The scheme A includes schemes A-1 to A-3, and the scheme B includes schemes B-1 to B-3.

### <Scheme A>

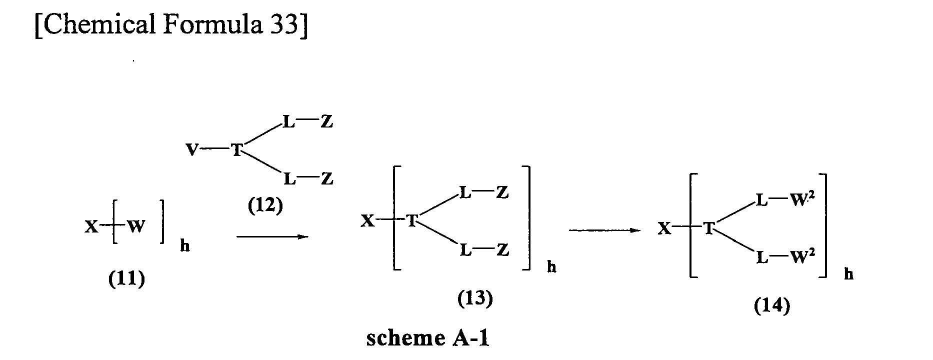

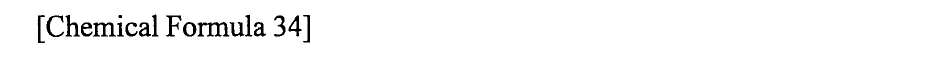

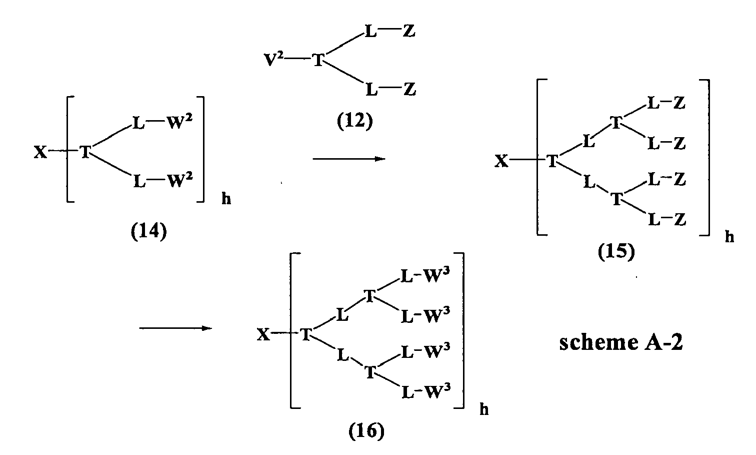

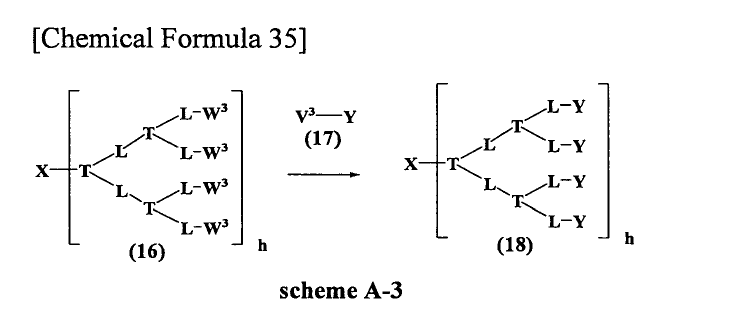

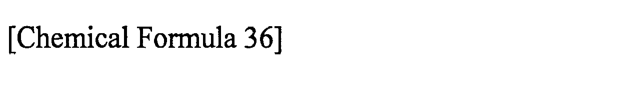

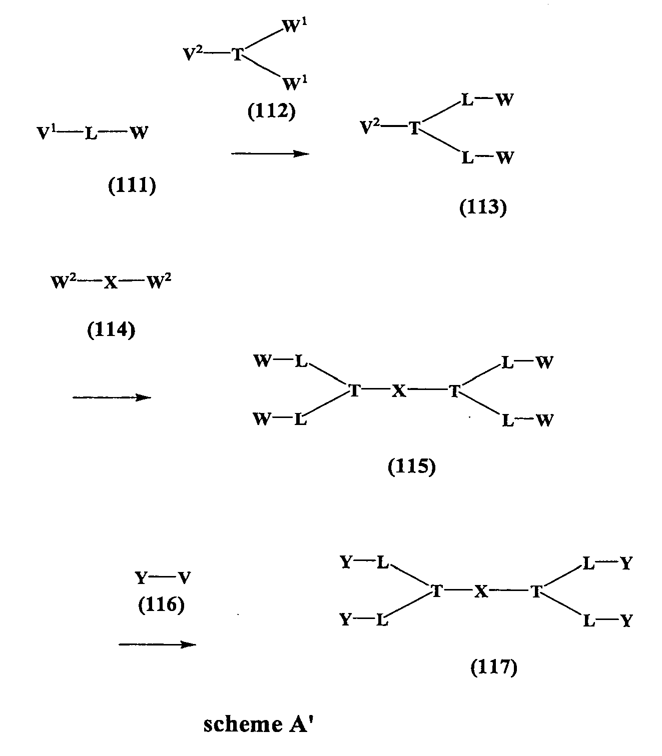

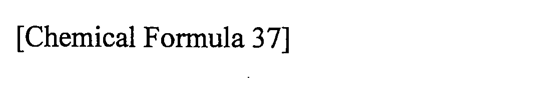

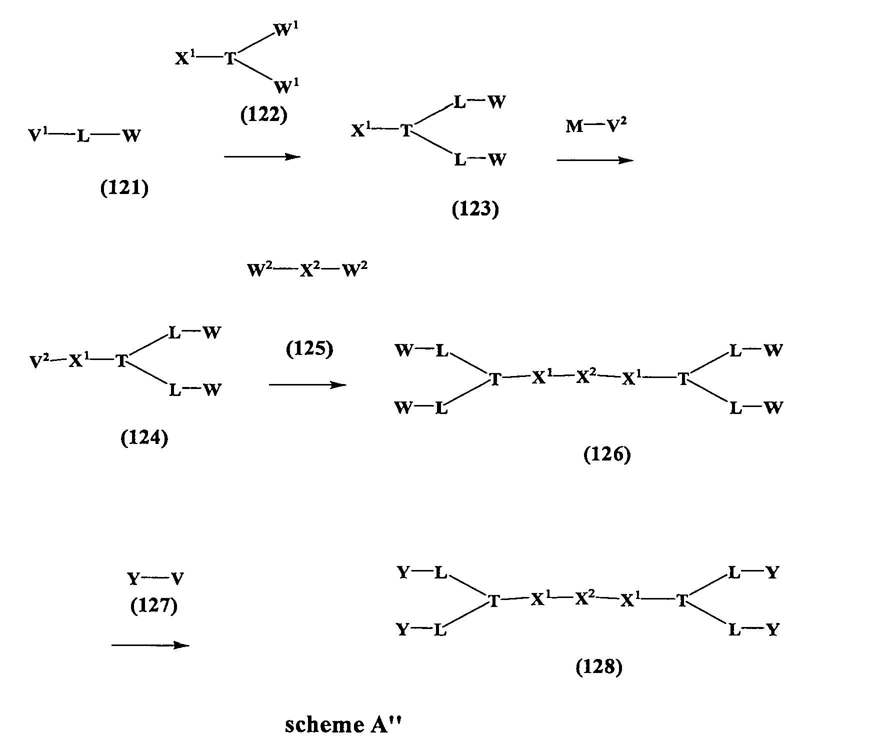

### <Scheme B>

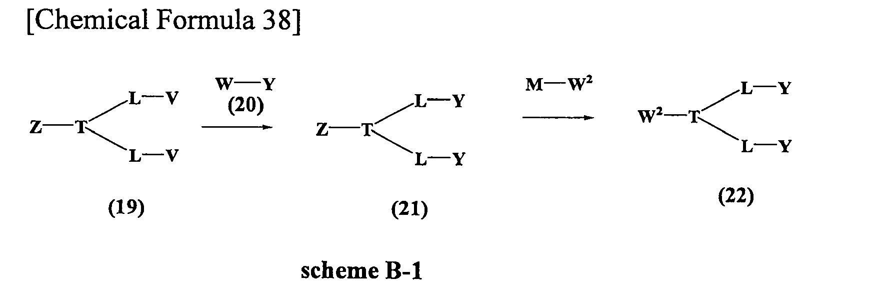

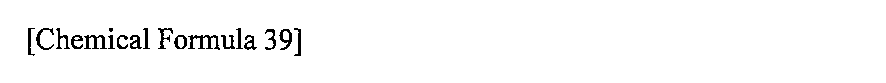

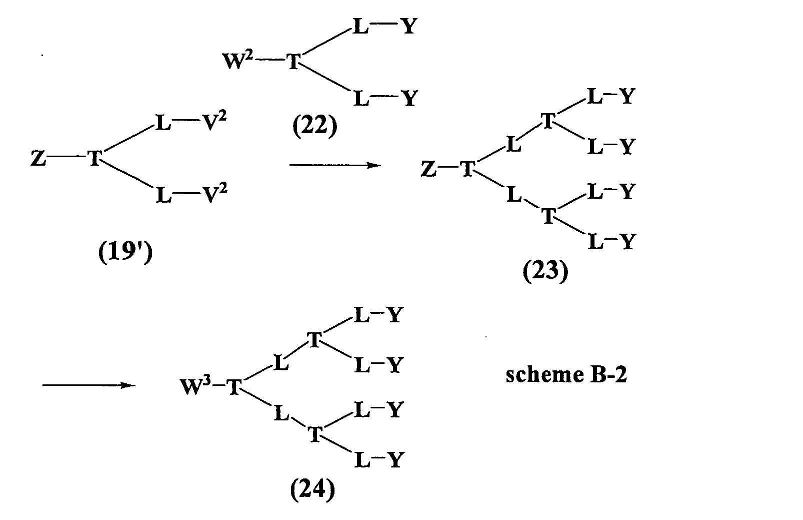

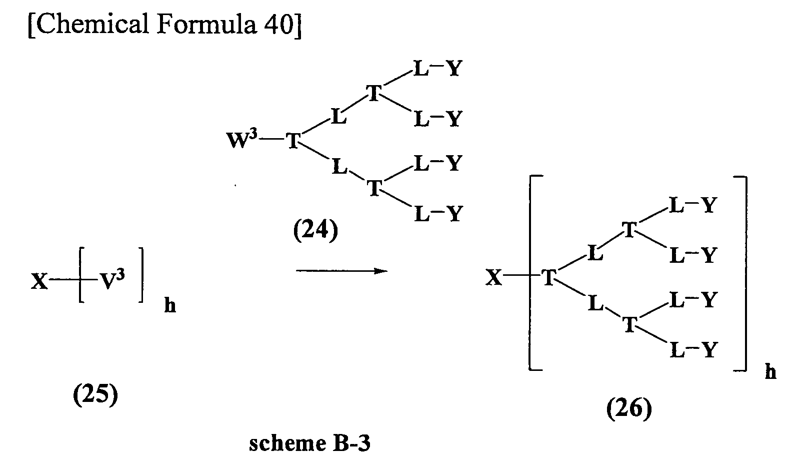

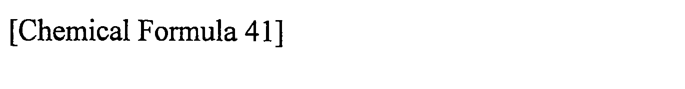

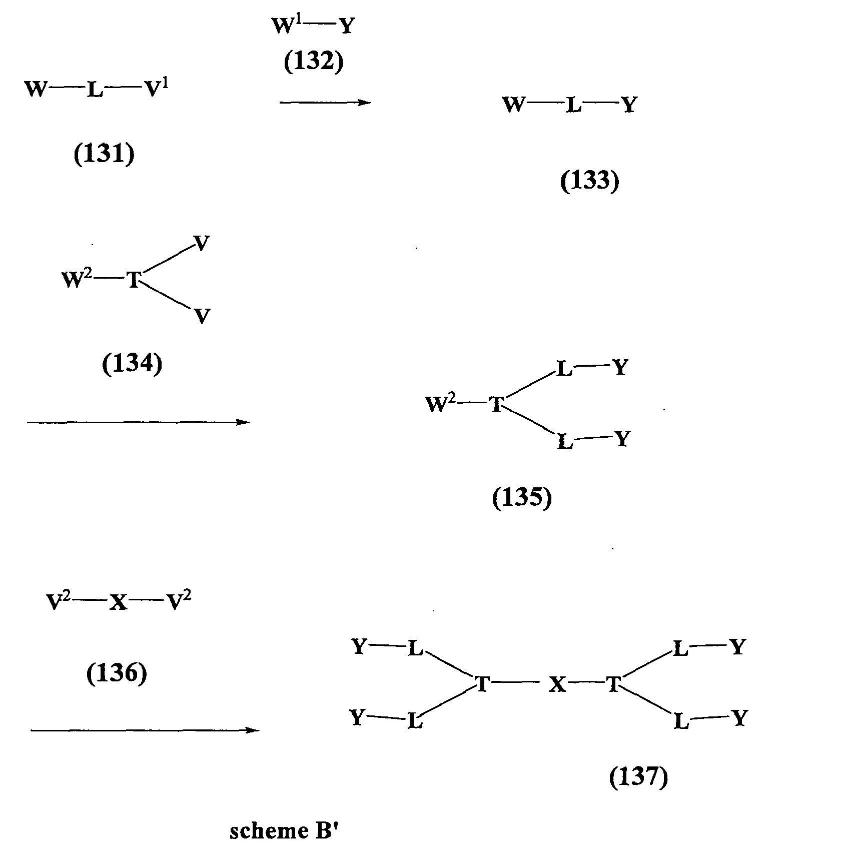

Here, X, T, L and Y are synonymous with the above description, and X¹ and X² represent a partial structure of X. W¹ to W³ (which is occasionally referred to as W) and V¹ to V³ (which is occasionally referred to as V) represent an active functional group which reacts with each other; M represents a hydrogen atom or an active functional group; and Z represents a protective group. In addition, h is an integer of 2 or more.

The schemes A and B illustrate examples in which there are two steps of introducing the repeating unit expressed by the above formula (1). Specifically, the schemes A and B illustrate a method for producing the branched compound which includes: a core part; and a side chain part that is formed from a repeating unit (referred to as a first generation) which is bonded to the core part and is expressed by the above formula (1), and a repeating unit (referred to as a second generation) which is bonded to the outer side thereof and is expressed by the above formula (1).

The generation number of the branched compound of the present invention may be one or more. It is preferable that the branched compound contains many numbers of the repeating unit which is expressed by the above formula (1), from the viewpoint of electric-charge transportability, and accordingly the generation number is preferably large, but the production process becomes long. The generation number is appropriately selected from the compactness and the ease of synthesis of the repeating unit which is expressed by the formula (1), the generation number is preferably 1 to 8, further preferably is 2 to 6, and particularly preferably is 2 to 3. The repeating units expressed by the formula (1) in each generation may be the same or different.

In addition, the branched compound of the present invention can be produced by using the following scheme C as well. The scheme C includes schemes C-1 to C-4.

### <scheme C>

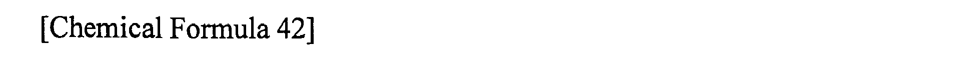

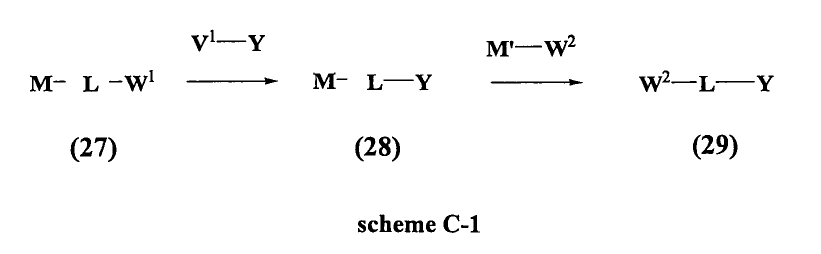

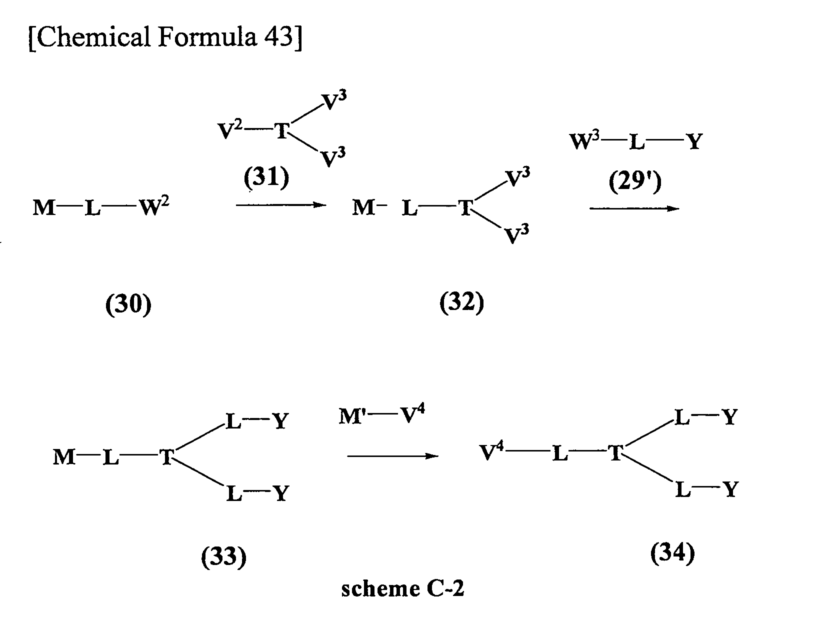

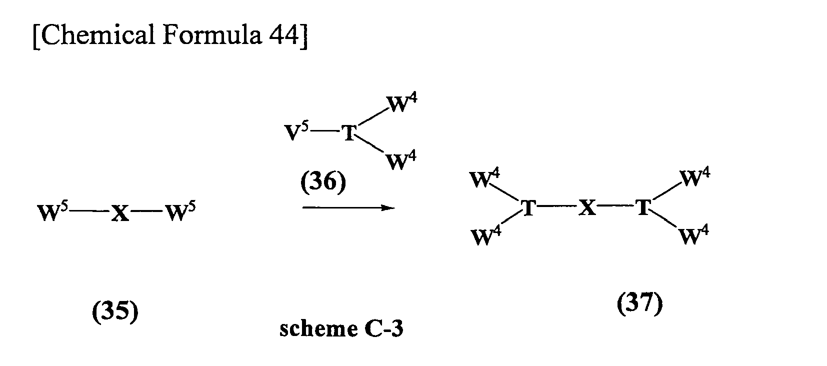

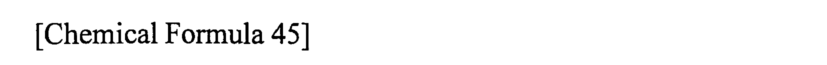

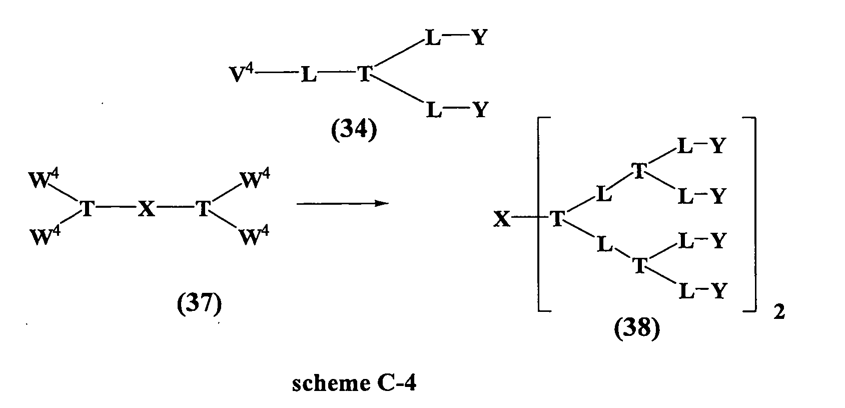

The branched compound can also be produced by the following scheme D with the use of a compound (34) obtained in the scheme C-2. The scheme D includes schemes D-1 and D-2.

### <scheme D>

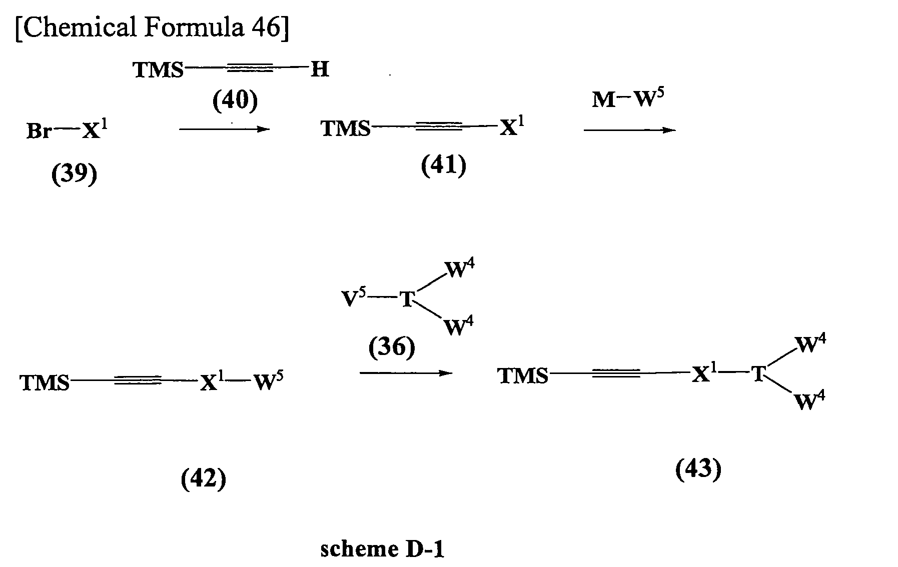

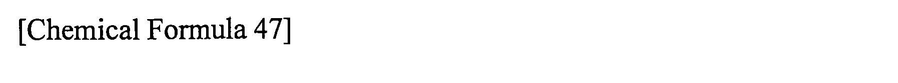

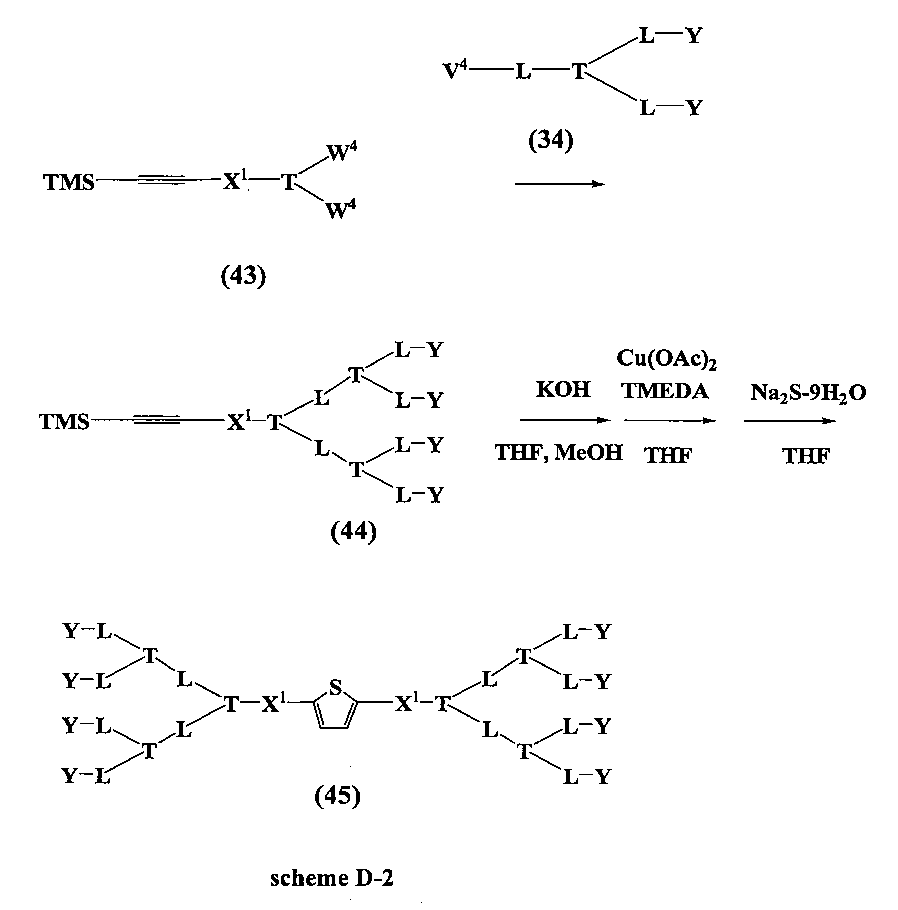

Here, X, T, L and Y are synonymous with the above description, and X¹ represents a partial structure of X. W¹ to W⁵ (which may be referred to as W) and V¹ to V⁵ (which may be referred to as V) represent active functional groups which react with each other, but functional groups having a lower reactivity between W and W than the reactivity between V and W are preferably selected. M and M' represent a hydrogen atom or an active functional group, and TMS is a protective group and represents a trimethyl silyl group.

When the branched compound is produced by the schemes C and D as well, the case of the generation number 2 is taken as an example, but the generation number can be set at 1 or more; the generation number may be appropriately selected from the compactness and the ease of synthesis of the repeating unit which is expressed by the formula (1); and the generation number is preferably 1 to 8, further preferably is 2 to 6, and particularly preferably is 2 to 3. The repeating units in each generation, which are expressed by the formula (1), may be the same or different.

The method for producing the branched compound of the present invention will be described more in detail below.

Processes for causing the reaction of bonding the active functional groups V and W include, for instance, a process of using a Suzuki coupling reaction, a process of using a Grignard reaction, a process of using a Stille reaction and a process of using a dehalogenation reaction.

Among these, the process of using the Suzuki coupling reaction and the process of using the Stille reaction are preferable from the availability of raw materials and the ease of the reaction operation.

In the case of the Suzuki coupling reaction, palladium [tetrakis (triphenyl phosphine)], palladium acetates or the like, for instance, is used as the catalyst; and an equivalent amount or more of an inorganic base such as potassium carbonate, sodium carbonate and barium hydroxide, an organic base such as triethyl amine and an inorganic salt such as cesium fluoride are added with respect to a monomer, and preferably the 1 to 10 equivalent amounts thereof are added to the monomer to cause the reaction. The inorganic salt may be made to be an aqueous solution and be reacted in a two-phase system. The examples of the solvent include N,N-dimethylformamide, toluene, dimethoxyethane and tetrahydrofuran. The reaction temperature is preferably approximately 50 to 160°C though depending on the solvent to be used. The reaction temperature may raise to approximately the boiling point of the solvent, and the solvent may be refluxed. The reaction time is approximately 1 hour to 200 hours. The Suzuki coupling reaction is described, for instance, in a Chemical Review (Chem. Rev.), vol. 95, p. 2457 (1995).

In the case of the Stille reaction, palladium [tetrakis (triphenyl phosphine)], palladium acetates or the like, for instance, is used as the catalyst, and an organotin compound is reacted as a monomer. The examples of the solvent include N,N-dimethylformamide, toluene, dimethoxyethane and tetrahydrofuran. The reaction temperature is preferably approximately 50 to 160°C though depending on the solvent to be used. The reaction temperature may raise to approximately the boiling point of the solvent, and the solvent may be refluxed. The reaction time is approximately 1 hour to 200 hours.

Examples of the active functional group include a halogen atom, an alkyl sulfonate group, an aryl sulfonate group, an aryl alkyl sulfonate group, a boric ester residue, a sulfonium methyl group, a phosphonium methyl group, a phosphonate methyl group, a monohalogenomethyl group, a boric acid residue, a formyl group, an alkyl stannyl group and a vinyl group; and the combination can be appropriately selected according to a reaction to be used and be used. The boric ester residue includes, for instance, a group expressed by the following formula.

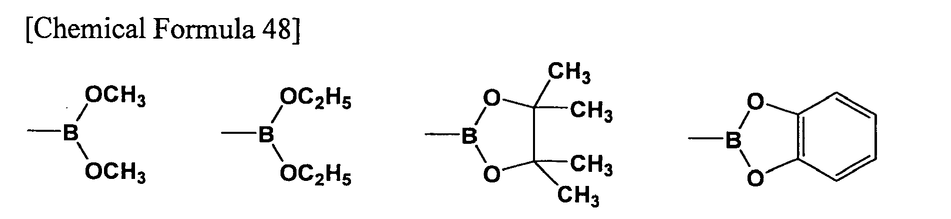

A combination of a halogen atom and a boric ester residue or a boric acid residue is preferable as the combination of the active functional groups V and W, for instance, in the process of using the Suzuki coupling reaction, and a combination of a halogen atom and an alkyl stannyl group is preferable in the process of using the Stille reaction.

A suitable group for the protective group may be selected according to a site to be protected and a reaction to be used, and a protective group described in "<Protective Groupes in Organic Syntehesis, 3rd ed. T.W. Greene and P.G.M.. Wuts, 1999 John Willey & Sons, Inc.>" is preferable. For instance, when a site to be protected is alkyne, the protective group is preferably a trialkyl silyl group such as a trimethyl silyl group, a triethyl silyl group and a t-butyl dimethyl silyl group, an aryl dialkyl silyl group such as a biphenyl dimethyl silyl group, and a 2-hydroxypropyl group, and the trimethyl silyl group is preferable.

The monomer to be reacted is dissolved in an organic solvent as needed, and can be reacted, for instance, at a temperature of the melting point or higher and the boiling point or lower of the organic solvent, with the use of an alkali or a suitable catalyst.

The organic solvent generally employs a solvent which has been subjected to a sufficient deoxygenation treatment so as to suppress a side reaction, though varying depending on a compound and reaction to be used, and it is preferable to progress the reaction in an inert atmosphere. The organic solvent is also preferably subjected to dehydration treatment (though this shall not be applied to the case of a reaction in a two-phase system containing water as in the Suzuki coupling reaction).

When the branched compound of the present invention is produced, an alkali or an appropriate catalyst can be appropriately added. These may be selected according to the reaction to be used. In addition, it is preferable that the above described alkali or catalyst to be used sufficiently dissolves in the solvent to be used for the reaction.

When the branched compound of the present invention is used as a material for an organic thin film device, its purity gives influence on device characteristics, so it is preferable to refine the monomer before the reaction with methods of distillation, sublimation refinement, recrystallization and the like, and then use the refined monomer for the reaction, and also preferable to subject the product obtained after the synthesis to refinement treatment such as sublimation refinement, recrystallization, reprecipitation refinement and classification by chromatography.

Examples of the solvent which is used for the reaction include a saturated hydrocarbon such as pentane, hexane, heptane, octane and cyclohexane, an unsaturated hydrocarbon such as benzene, toluene, ethylbenzene and xylene, a halogenated saturated hydrocarbon such as carbon tetrachloride, chloroform, dichloromethane, chlorobutane, bromobutane, chloropentane, bromopentane, chlorohexane, bromohexane, chlorocyclohexane and bromocyclohexane, a halogenated unsaturated hydrocarbon such as chlorobenzene, dichlorobenzene and trichlorobenzene, alcohols such as methanol, ethanol, propanol, isopropanol, butanol and t-butyl alcohol, carboxylic acids such as formic acid, acetic acid and propionic acid, ethers such as dimethyl ether, diethyl ether, methyl-t-butyl ether, tetrahydrofuran, tetrahydropyran and dioxane, and an inorganic acid such as hydrochloric acid, bromic acid, hydrofluoric acid, sulfuric acid and nitric acid; and a single solvent or a mixture solvent of these may be used.

A product can be obtained by subjecting the product obtained after the reaction to a normal post-treatment, for instance, of quenching the product in water, extracting the product with an organic solvent and distilling the solvent off. The isolation and refinement of the product can be conducted by a method of preparative isolation by chromatography, recrystallization and the like.

Next, an organic thin film of the present invention will be described below. The organic thin film of the present invention contains the above described branched compound of the present invention.

The film thickness of the organic thin film is usually approximately 1 nm to 100 µm, preferably is 2 nm to 1,000 nm, further preferably is 5 nm to 500 nm, and particularly preferably is 20 nm to 200 nm.

The organic thin film may contain solely one type of the above described branched compound, and may also contain two or more types of the above described branched compound. In addition, a low-molecular compound or a high-molecular compound other than the above described branched compound, which has electron transportability or hole transportability, can also be used in a form of being mixed, in order to enhance the electron transportability or the hole transportability of the organic thin film.

A well-known material can be used as the hole transportable material, and the examples include, for instance, a pyrazoline derivative, an arylamine derivative, a stilbene derivative, a triaryl diamine derivative, oligothiophene and a derivative thereof, polyvinyl carbazole and a derivative thereof, polysilane and a derivative thereof, a polysiloxane derivative having an aromatic amine in a side chain or a main chain, polyaniline and a derivative thereof, polythiophene and a derivative thereof, polypyrrole and a derivative thereof, polyarylene vinylene and a derivative thereof, and polythienylene vinylene and a derivative thereof. A well-known material can be used as the electron transportable material, and the examples include an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyl dicyanoethylene and a derivative thereof, a diphenoquinone derivative, 8-hydroxyquinoline and a metal complex of a derivative thereof, polyquinoline and a derivative thereof, polyquinoxaline and a derivative thereof, polyfluorene and a derivative thereof, and fullerenes such as C₆₀ and a derivative thereof.

The organic thin film of the present invention may contain a charge-generating material so as to generate an electric charge due to a light absorbed in the organic thin film. A well-known material can be used as the charge-generating material, and the examples include an azo compound and a derivative thereof, a diazo compound and a derivative thereof, a non-metallic phthalocyanine compound and a derivative thereof, a metallic phthalocyanine compound and a derivative thereof, a perylene compound and a derivative thereof, a polycyclic quinone-based compound and a derivative thereof, a squarylium compound and a derivative thereof, an azulenium compound and a derivative thereof, a thiapyrylium compound and a derivative thereof, and fullerenes such as C₆₀ and a derivative thereof.

Furthermore, the organic thin film of the present invention may contain a material necessary for developing various functions, and may contain, for instance, a sensitizer for sensitizing a function of generating an electric charge due to the absorbed light, a stabilizer for enhancing stability, a UV absorber for absorbing UV light, and the like.

The organic thin film of the present invention may contain a high-molecular compound material other than the above described branched compound as a high-molecular binder, in order to enhance mechanical characteristics. A high-molecular binder which does not extremely obstruct electron transportability or hole transportability is preferable and a high-molecular binder which does not strongly absorb a visible light is preferably used.

Examples of such a high-molecular binder include poly (N-vinylcarbazole), polyaniline and a derivative thereof, polythiophene and a derivative thereof, poly(p-phenylene vinylene) and a derivative thereof, poly(2,5-thienylene vinylene) and a derivative thereof, polycarbonate, polyacrylate, polymethyl acrylate, polymethyl methacrylate, polystyrene, polyvinyl chloride and polysiloxane.

Examples of a method for producing the organic thin film of the present invention include, for instance, a method of forming a film from a solution containing the above described branched compound, an electron transportable material or a hole transportable material, which is mixed as needed, and a high-molecular binder. The thin film of the branched compound of the present invention can be formed also with a vacuum deposition method.

A solvent which is used for forming the film from the solution may be a solvent which dissolves the branched compound, the electron transportable material or the hole transportable material to be mixed, and the high-molecular binder therein.

Examples of the solvent to be used when the organic thin film of the present invention is formed from the solution include an unsaturated hydrocarbon-based solvent such as toluene, xylene, mesitylene, tetralin, decalin, bicyclohexyl, n-butylbenzene, sec-butylbenzene and tert-butylbenzene, a halogenated saturated hydrocarbon-based solvent such as carbon tetrachloride, chloroform, dichloromethane, dichloroethane, chlorobutane, bromobutane, chloropentane, bromopentane, chlorohexane, bromohexane, chlorocyclohexane and bromocyclohexane, a halogenated unsaturated hydrocarbon-based solvent such as chlorobenzene, dichlorobenzene and trichlorobenzene, and an ethers-based solvent such as tetrahydrofuran and tetrahydropyran. These solvents can ordinarily dissolve 0.1 mass% or more of the branched compound therein though depending on its structure and the molecular weight of the branched compound.

For forming the film from the solution, it is possible to use an application method such as a spin coating method, a casting method, a microgravure coating method, a gravure coating method, a bar coating method, a roll coating method, a wire bar coating method, a dip coating method, a spray coating method, a screen printing method, a flexo printing method, an offset printing method, an ink jet printing method and a dispenser printing method; and it is preferable to use the spin coating method, the flexo printing method, the ink jet printing method and the dispenser printing method.

The organic thin film of the present invention is preferably subjected to an annealing treatment after the film has been formed. By the annealing treatment, the interaction between the branched compounds is promoted, thereby the film quality of the organic thin film is improved, and electron mobility or hole mobility enhances. The treatment temperature of the annealing treatment is preferably a temperature in the vicinity between 50°C and a glass transition temperature (Tg) of the branched compound, and more preferably is a temperature between (Tg-30°C) and Tg. The time for the annealing treatment is preferably in a range of 1 minute to 10 hours, and more preferably is in a range of 10 minutes to 1 hour. An atmosphere for the annealing treatment is preferably in a vacuum or in an inert gas atmosphere.

The organic thin film of the present invention has electron transportability or hole transportability, and accordingly can be used for various organic thin film devices such as an organic thin film transistor, an organic thin-film light-emitting transistor, an organic solar cell and a photosensor, by controlling the transportation of electrons or holes injected from an electrode, or electric charges generated by light absorption.

The organic thin film of the present invention has the bipolarity of the hole transportability and the electron transportability, and can be used for various organic thin film devices such as an organic electroluminescence device, an organic thin film transistor, an organic thin-film light-emitting transistor, an organic solar cell and a photosensor, by controlling the transportation of holes injected from an electrode, holes or electrons generated by light absorption.

### (Organic thin film transistor)

Firstly, the organic thin film transistor according to preferred embodiments will be described below. The organic thin film transistor may have a structure having a source electrode and a drain electrode, an organic thin-film layer (active layer) which becomes a current path between the source electrode and the drain electrode and contains the branched compound of the present invention, and a gate electrode which controls current quantity passing through the current path, and the examples include an electric-field effect type and an electrostatic induction type.

The electric-field effect type organic thin film transistor preferably has a source electrode and a drain electrode, an organic thin film layer (active layer) which becomes a current path between the source electrode and the drain electrode and contains the branched compound of the present invention, a gate electrode which controls the current quantity passing through the current path, and an insulation layer arranged between the active layer and the gate electrode. It is particularly preferable that the source electrode and the drain electrode are provided in contact with the organic thin-film layer (active layer) containing the branched compound of the present invention, and that the gate electrode is further provided so as to sandwich the insulation layer which comes in contact with the organic thin-film layer.

It is preferable that the electrostatic induction type organic thin film transistor has a source electrode and a drain electrode, an organic thin-film layer which becomes a current path between the source electrode and the drain electrode and contains the branched compound of the present invention and a gate electrode which controls the current quantity passing through the current path, and that the gate electrode is preferably provided in the organic thin-film layer. It is particularly preferable that the source electrode, the drain electrode and the gate electrode provided in the organic thin-film layer are provided in contact with the organic thin-film layer containing the branched compound of the present invention. The gate electrode may have a structure in which a current path through which an electric current passes from the source electrode to the drain electrode is formed and the quantity of the electric current that passes through the current path can be controlled by voltage applied to the gate electrode, and includes a comb-shaped electrode, for instance.

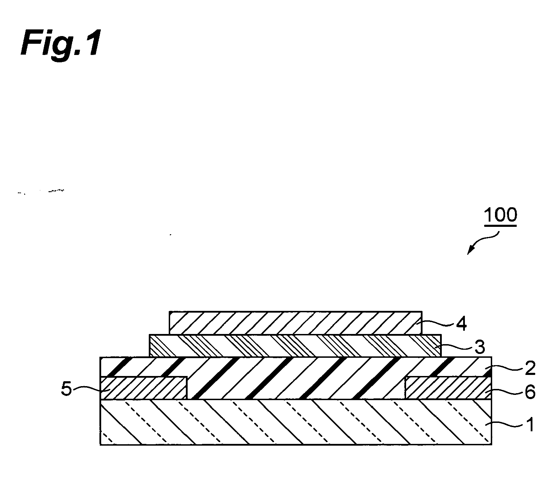

Figure 1 is a schematic sectional view of an organic thin film transistor (electric-field effect type organic thin film transistor) according to a first embodiment. The organic thin film transistor 100 illustrated in Figure 1 includes: a substrate 1; a source electrode 5 and a drain electrode 6 formed on the substrate 1 so as to have a predetermined space; an active layer 2 formed on the substrate 1 so as to cover the source electrode 5 and the drain electrode 6; an insulation layer 3 formed on the active layer 2; and a gate electrode 4 formed on the insulation layer 3 so as to cover a region of the insulation layer 3 between the source electrode 5 and the drain electrode 6.

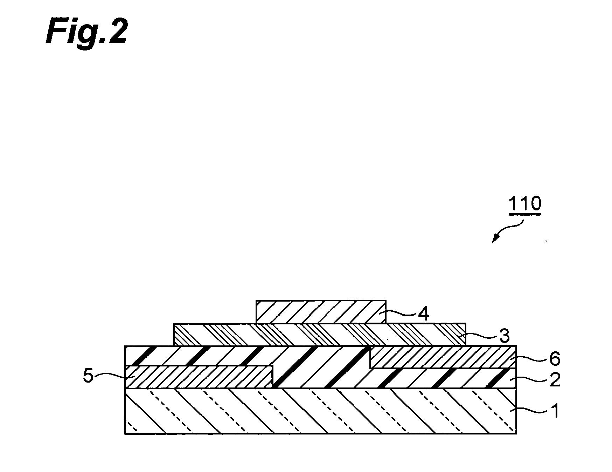

Figure 2 is a schematic sectional view of an organic thin film transistor (electric-field effect type organic thin film transistor) according to a second embodiment. The organic thin film transistor 110 illustrated in Figure 2 includes: a substrate 1; a source electrode 5 formed on the substrate 1; an active layer 2 formed on the substrate 1 so as to cover the source electrode 5; a drain electrode 6 formed on the active layer 2 so as to have a predetermined space to the source electrode 5; an insulation layer 3 formed on the active layer 2 and the drain electrode 6; and a gate electrode 4 formed on the insulation layer 3 so as to cover a region of the insulation layer 3 between the source electrode 5 and the drain electrode 6.

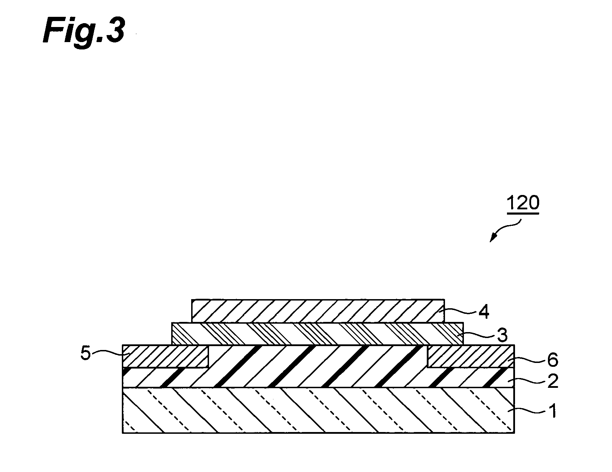

Figure 3 is a schematic sectional view of an organic thin film transistor (electric-field effect type organic thin film transistor) according to a third embodiment. The organic thin film transistor 120 illustrated in Figure 3 includes: a substrate 1; an active layer 2 formed on the substrate 1; a source electrode 5 and a drain electrode 6 formed on the active layer 2 so as to have a predetermined space; an insulation layer 3 formed on the active layer 2 so as to partially cover the source electrode 5 and the drain electrode 6; and a gate electrode 4 formed on the insulation layer 3 so as to partially cover each of a region of the insulation layer 3 having the source electrode 5 formed in its lower part and a region of the insulation layer 3 having the drain electrode 6 formed in its lower part.

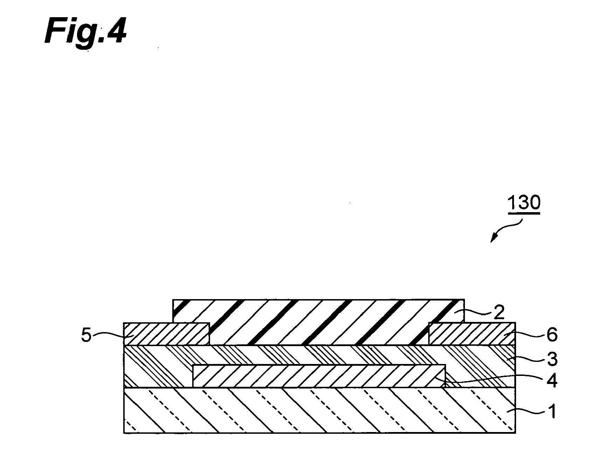

Figure 4 is a schematic sectional view of an organic thin film transistor (electric-field effect type organic thin film transistor) according to a fourth embodiment. The organic thin film transistor 130 illustrated in Figure 4 includes: a substrate 1; a gate electrode 4 formed on the substrate 1; an insulation layer 3 formed on the substrate 1 so as to cover the gate electrode 4; a source electrode 5 and a drain electrode 6 formed on the insulation layer 3 at a predetermined space so as to partially cover a region of the insulation layer 3 having the gate electrode 4 formed in its lower part; and an active layer 2 formed on the insulation layer 3 so as to partially cover the source electrode 5 and the drain electrode 6.

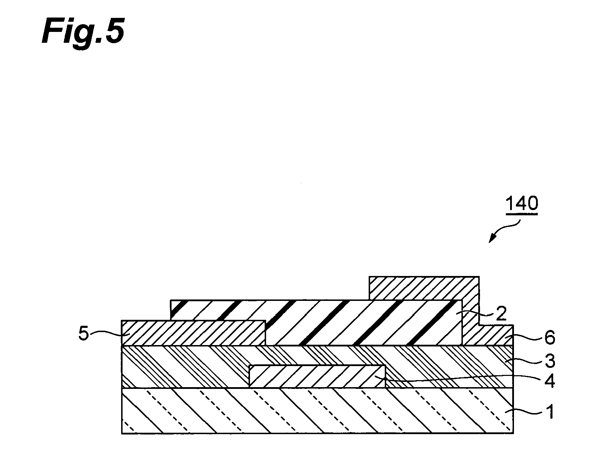

Figure 5 is a schematic sectional view of an organic thin film transistor (electric-field effect type organic thin film transistor) according to a fifth embodiment. The organic thin film transistor 140 illustrated in Figure 5 includes: a substrate 1; a gate electrode 4 formed on the substrate 1; an insulation layer 3 formed on the substrate 1 so as to cover the gate electrode 4; a source electrode 5 formed on the insulation layer 3 so as to partially cover a region of the insulation layer 3 having the gate electrode 4 formed in its lower part; an active layer 2 formed on the insulation layer 3 so as to partially cover the source electrode 5; and a drain electrode 6 formed on the insulation layer 3 so as to partially cover a region of the active layer 2 having the gate electrode 4 formed in its lower part and have a predetermined space to the source electrode 5.

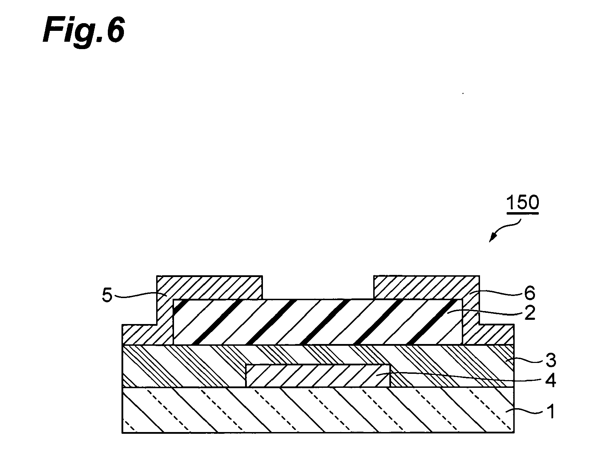

Figure 6 is a schematic sectional view of an organic thin film transistor (electric-field effect type organic thin film transistor) according to a sixth embodiment. The organic thin film transistor 150 illustrated in Figure 6 includes: a substrate 1; a gate electrode 4 formed on the substrate 1; an insulation layer 3 formed on the substrate 1 so as to cover the gate electrode 4; an active layer 2 formed so as to cover a region of the insulation layer 3 having the gate electrode 4 formed in its lower part; a source electrode 5 formed on the insulation layer 3 so as to partially cover a region of the active layer 2 having the gate electrode 4 formed in its lower part; and a drain electrode 6 formed on the insulation layer 3 so as to have a predetermined space to the source electrode 5 and partially cover a region of the active layer 2 having the gate electrode 4 formed in its lower part.

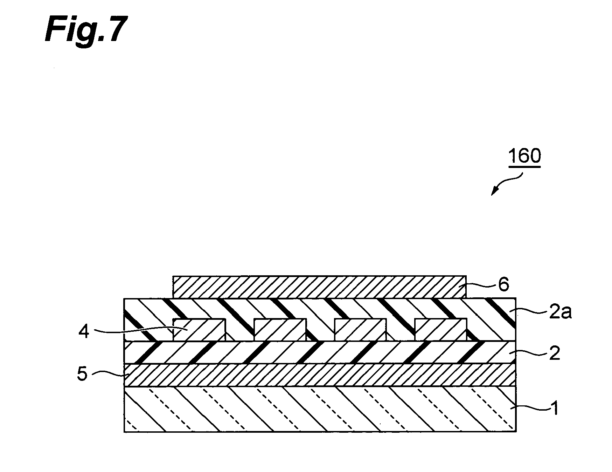

Figure 7 is a schematic sectional view of an organic thin film transistor (electrostatic induction type organic thin film transistor) according to a seventh embodiment. The organic thin film transistor 160 illustrated in Figure 7 includes: a substrate 1; a source electrode 5 formed on the substrate 1; an active layer 2 formed on the source electrode 5; a plurality of gate electrodes 4 formed on the active layer 2 so as to have a predetermined space; an active layer 2a (where the material constituting the active layer 2a may be the same as or different from that of the active layer 2) formed on the active layer 2 so as to cover the whole of the gate electrode 4; and a drain electrode 6 formed on the active layer 2a.

In the organic thin film transistor according to the first to the seventh embodiments, the active layer 2 and/or the active layer 2a contain the branched compound of the present invention, and become a current path (channel) between the source electrode 5 and the drain electrode 6. The gate electrode 4 controls the current quantity passing through the current path (channel) in the active layer 2 and/or the active layer 2a by the application of voltage.

Such an electric-field effect type organic thin film transistor can be produced with a well-known method, for instance, a method described in Japanese Patent Application Laid-Open Publication No. 5-110069. An electrostatic induction type organic thin film transistor can be produced with a well-known method, for instance, a method described in Japanese Patent Application Laid-Open Publication No. 2004-006476.

The substrate 1 may be any type as long as the substrate does not obstruct characteristics of the organic thin film transistor, and can employ a glass substrate, a flexible film substrate and a plastic substrate.

It is extremely advantageous and preferable in production to use an organic-solvent-soluble compound when forming the active layer 2, and accordingly, the organic thin film to be active layer 2 can be formed by using a method described above for producing the organic thin film of the present invention.

A material of the insulation layer 3 contacting the active layer 2 may be a material having high electric insulation properties, and can employ a well-known material. The material of the insulation layer 3 includes, for instance, SiOₓ, SiNx, Ta₂O₅, polyimide, polyvinyl alcohol, polyvinyl phenol and organic glass. A material having a high dielectric constant is more preferable from the viewpoint of lowering voltage.

When forming the active layer 2 on the insulation layer 3, it is also possible to modify the surface of the insulation layer 3 by treating the surface with a surface treatment agent such as a silane coupling agent and then form the active layer 2, in order to enhance interfacial characteristics between the insulation layer 3 and the active layer 2. The surface treatment agent includes long-chain alkylchlorosilanes, long-chain alkyl alkoxy silanes, fluorination alkylchlorosilanes, fluorinated alkyl alkoxy silanes, and a silylamine compound such as hexamethyldisilazane. It is also possible to treat the surface of the insulation layer with ozone UV or O₂ plasma before treating the surface with the surface treatment agent.

It is preferable to form a protective film on the organic thin film transistor in order to protect the device after having produced the organic thin film transistor. Thereby, the organic thin film transistor is blocked from the atmosphere, which can suppress the degradation of the characteristics of the organic thin film transistor. In addition, the protective film can decrease the influence when the display device to be driven is formed on the organic thin film transistor.

The method for forming the protective film includes a method of covering the device with a UV curable resin, a thermoset resin, an inorganic SiONx film or the like. It is preferable for effectively blocking the device from the atmosphere to conduct a step until forming the protective film after having formed the organic thin film transistor, without exposing the device to the atmosphere (in dried nitrogen atmosphere or in the vacuum, for instance).

The organic thin film transistor of the present invention uses a branched compound which functions as a bipolar organic semiconductor for its active layer, and thereby can be used as an organic thin-film light-emitting transistor as well.

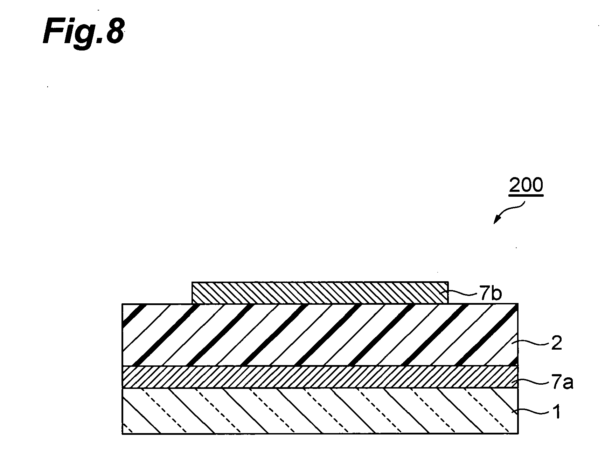

Next, the application of the organic thin film of the present invention to a solar cell will be described below. Figure 8 is a schematic sectional view of the solar cell according to an embodiment. The solar cell 200 illustrated in Figure 8 includes: a substrate 1; a first electrode 7a formed on the substrate 1; an active layer 2 which is formed of an organic thin film containing the branched compound of the present invention and is formed on the first electrode 7a; and a second electrode 7b formed on the active layer 2.

The solar cell according to the present embodiment uses a transparent or translucent electrode in one of the first electrode 7a and the second electrode 7b. The electrode material can use a metal such as aluminum, gold, silver, copper, an alkaline metal and an alkaline-earth metal, or a translucent film and a transparent electroconductive film thereof. In order to obtain a high open voltage, it is preferable that each of the electrodes is selected so as to have a large difference between the work functions. The active layer 2 (organic thin film) can employ a carrier-generating agent, a sensitizer and the like which are added, in order to enhance the light sensitivity. For a substrate 1, a silicon substrate, a glass substrate, a plastic substrate or the like can be used.

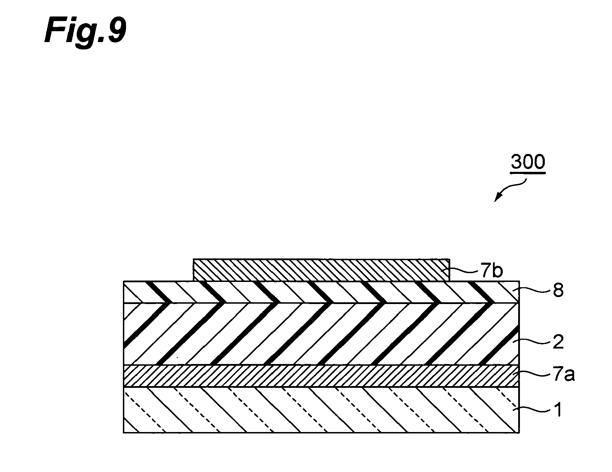

Next, the application of the organic thin film of the present invention to a photosensor will be described below. Figure 9 is a schematic sectional view of a photosensor according to a first embodiment. The photosensor 300 illustrated in Figure 9 includes: a substrate 1; a first electrode 7a formed on the substrate 1; an active layer 2 which is formed of an organic thin film containing the branched compound of the present invention and is formed on the first electrode 7a; a charge-generating layer 8 formed on the active layer 2; and a second electrode 7b formed on the charge-generating layer 8.

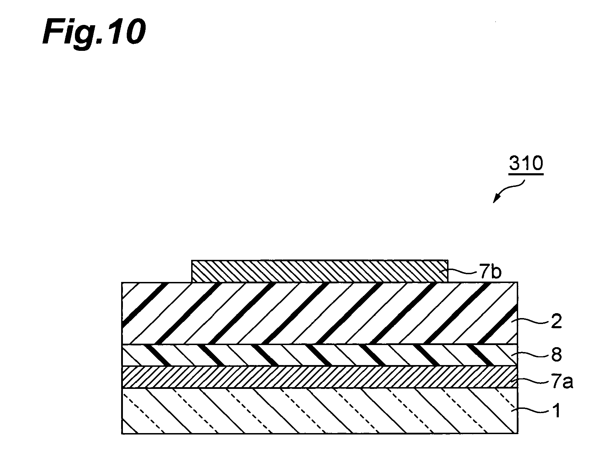

Figure 10 is a schematic sectional view of a photosensor according to a second embodiment. The photosensor 310 illustrated in Figure 10 includes: a substrate 1; a first electrode 7a formed on the substrate 1; a charge-generating layer 8 formed on the first electrode 7a; an active layer 2 which is formed of an organic thin film containing the branched compound of the present invention and is formed on the charge-generating layer 8; and a second electrode 7b formed on the active layer 2.

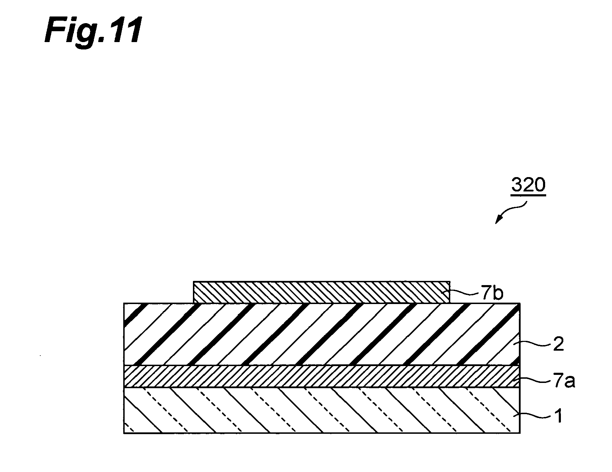

Figure 11 is a schematic sectional view of a photosensor according to a third embodiment. The photosensor 320 illustrated in Figure 11 includes: a substrate 1; a first electrode 7a formed on the substrate 1; an active layer 2 which is formed of an organic thin film containing the branched compound of the present invention and is formed on the first electrode 7a; and a second electrode 7b formed on the active layer 2.

In the photosensor according to the first to third embodiments, a transparent or translucent electrode is used for one of the first electrode 7a and the second electrode 7b. The charge-generating layer 8 is a layer which absorbs light to generate an electric charge. The electrode material can use a metal such as aluminum, gold, silver, copper, an alkaline metal and an alkaline-earth metal, or a translucent film and a transparent electroconductive film thereof. The active layer 2 (organic thin film) can employ a carrier-generating agent, a sensitizer and the like which are added, in order to enhance the light sensitivity. For a substrate 1, a silicon substrate, a glass substrate, a plastic substrate or the like can be used.

### Examples

The present invention will be described more specifically below with reference to examples and comparative examples, but the present invention is not limited at all to the examples described below.

### (Measurement condition or the like)

A nuclear magnetic resonance (NMR) spectrum was measured by using a trade name JMN-270 made by JEOL (JEOL Ltd.) (270 MHz when measuring ¹H) or a trade name JMNLA-600 made by the same company (150 MHz when measuring ¹³C). The chemical shift is expressed by parts per million (ppm). Tetramethylsilane (TMS) was used for 0 ppm of an internal standard. A coupling constant (J) is expressed by hertz, and abbreviations s, d, t, m and br represent a single line (singlet), a double line (doublet), a triple line (triplet), a quadruple line (quartet), a multiple line (multiplet) and a broad width line (broad), respectively. Mass spectrometry (MS) was measured by using Voyager Linear DE-H MALDI-TOF MS (trade name) made by PerSeptive Biosystems. Silica gel in a column chromatographic separation employed a trade name Silicagel 60N (40 to 50 µm) made by Kanto Chemical Co., Inc. Alumina employed a trade name aluminium oxide 90 standardized made by Merck & Co.,. All chemicals are reagent grades, and were purchased from Wako Pure Chemical Industries, Ltd., Tokyo Chemical Industry Co., Ltd., Kanto Chemical Co., Inc., INC., Nacalai Tesque, Inc. and Sigma-Aldrich Japan K.K.

Cyclic voltammetry employed an apparatus made by BAS, and was measured by using a Pt electrode made by BAS Inc., as a working electrode, using a Pt wire as a counter electrode, and using an Ag wire as a reference electrode. A sweep rate in this measurement was 100 mV/sec, and a scanning potential region was -2.8V to 1.6V. The reduction potential and the oxidation potential were measured by completely dissolving 1×10⁻³ mol/L of the compound and 0.1 mol/L of tetrabutylammonium hexafluorophosphate (TBAPF6) which is a supporting electrolyte into a methylene chloride solvent.

### (Referential example 1 of synthesis)

### <Synthesis of SnBu₃-4T-SnBu₃>

Into a 30 mL two-neck flask of which the inside had been heated, dried and replaced with nitrogen, 5,5"'-dibromo-3,3"'-dihexyl-quarter thiophene (Br-4T-Br) (230 mg, 0.35 mmol) was charged, dried THF (3 mL) was added thereto, subsequently, the inside was cooled to - 78°C, and 1.6 M n-butyl lithium / hexane (0.66 mL, 1.05 mmol) was added dropwise. After the liquid was stirred for 30 minutes, tributyltin chloride (0.25 mL, 1.4 mmol) was added at a time. The reaction system was heated to room temperature and was stirred for 3 hours. Next, water (1 mL) and hexane (20 mL) were added to the reaction solution. After an organic layer was washed twice by using water (20 mL), the product was dried by anhydrous sodium sulfate. A target object (SnBu₃-4T-SnBu₃) (360 mg, yield of 96%) expressed by the following formula was obtained as a yellow oil, by decompression-concentrating the product and then refining the concentrate with column chromatography (alumina, hexane).

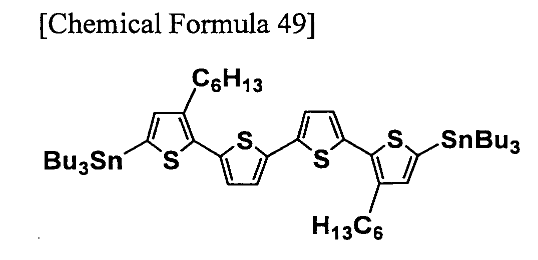

### (Referential example 2 of synthesis)

### <Synthesis of Ph-4T-SnBu₃>

Into a 50 mL two-neck flask of which the inside had been replaced with nitrogen, 5-bromo-3,3"'-dihexyl-quarter thiophene (4T-Br) (1.30 g, 2.25 mmol), phenylboronic acid (410 mg, 3.38 mmol), sodium carbonate (715 mg, 6.75 mmol) and tetrakistriphenyl phosphine palladium (0) (130 mg, 0.11 mmol) were charged, and DME (10 mL) and a purified water (1 mL) were added thereto. After the liquid was heated and refluxed for 12 hours, a solid material was removed with sellite filtration, and water (20 mL) and hexane (20 mL) were added. An organic layer was washed twice with water (20 mL), and the product was dried by anhydrous magnesium sulfate. A target object (4T-Ph) (1.2 g, yield of 93%) was obtained as a yellow oil, by removing an insoluble matter by filtration, then decompression-concentrating the product and refining the concentrate with column chromatography (silica gel, hexane).

Into a 50 mL two-neck flask of which the inside had been heated, dried and replaced with nitrogen, 4T-Ph (0.8 g, 1.4 mmol) obtained in the above description was charged, dried THF (14 mL) was added thereto, subsequently, the inside was cooled to -78°C, and 1.6 M n-butyl lithium / hexane (1.7 mL, 2.8 mmol) was added dropwise. After the liquid was stirred for 30 minutes, tributyltin chloride (0.75 mL, 4.2 mmol) was added at a time. The reaction system was heated to room temperature and was stirred for 3 hours. Water (20 mL) and hexane (20 mL) were added to the reaction solution, an organic layer was washed twice with water (20 mL), and the product was dried by anhydrous magnesium sulfate. A target object (Ph-4T-SnBu₃) (1.32 g, yield of 93%) was obtained as a yellow oil, by removing an insoluble matter by filtration, then decompression-concentrating the product and refining the concentrate with column chromatography(alumina, hexane/dichloromethane = 9/1).

### (Referential example 3 of synthesis)

### <Ph-8T-SnBu₃>

Into a 100 mL flask of which the inside had been replaced with nitrogen, 3,3"'-dihexyl-quarter thiophene (4T) (1.37 g, 2.75 mmol) and THF (20 mL) were charged, subsequently, the inside was cooled to 0°C, NBS (514 mg, 2.88 mmol) was added thereto, and the liquid was stirred for 3 hours. Subsequently, water (2 mL) was added to the reaction solution, and the reaction was stopped. After an organic layer was washed twice by using 20 mL of water, the product was dried by anhydrous sodium sulfate. A target object (4T-Br) (915 mg, yield of 57%) was obtained as a yellow oil, by decompression-concentrating the product and then refining the concentrate with column chromatography (silica gel, hexane).

Into a 30 mL flask of which the inside had been replaced with nitrogen, Ph-4T-SnBu₃ (300 mg, 0.347 mmol) and 4T-Br (300 mg, 0.52 mmol) were charged, and dried toluene (5 mL) was added thereto. After the liquid was degassed, tetrakistriphenyl phosphine palladium (0) (18 mg, 0.017 mmol) was added thereto, and the liquid was heated and refluxed for 12 hours. A target object (Ph-8T) (301 mg, yield of 81%) was obtained as a red oil, by decompression-concentrating the product and refining the concentrate with column chromatography (silica gel, hexane).

Into a 30 mL two-neck flask of which the inside had been heated, dried and replaced with nitrogen, Ph-8T (131 mg, 0.123 mmol) was charged, dried THF (14 mL) was added thereto, subsequently, the inside was cooled to -78°C, and 1.6 M n-butyl lithium / hexane (0.23 mL, 0.37 mmol) was added dropwise. After the liquid was stirred for 30 minutes, tributyltin chloride (0.13 mL, 0.49 mmol) was added at a time. The reaction system was heated to room temperature and was stirred for 3 hours. Water (20 mL) and hexane (20 mL) were added to the reaction solution. After an organic layer was washed twice by using water (20 mL), a yellow oil containing a target object (Ph-8T-SnBu₃) was obtained.

### (Referential example 4 of synthesis)

### <Synthesis of 4T-SnBu₃>

Into a 100 mL three-neck flask of which the inside had been heated, dried and replaced with nitrogen, 4T (1.98 g, 3.97 mmol) was charged. Dried THF (40 mL) was added to the liquid, subsequently, the liquid was cooled to -78°C, and 1.6 M n-butyl lithium / hexane (2.5 mL, 4.0 mmol) was added dropwise. After the liquid was stirred for 30 minutes, tributyltin chloride (1.2 mL, 4.4 mmol) was added at a time. The reaction system was heated to room temperature and was stirred for 3 hours. Water (20 mL) and hexane (20 mL) were added to the reaction solution. After an organic layer was washed twice with water (20 mL), the product was dried by anhydrous magnesium sulfate. A target object (4T-SnBu₃) (1.64 g, yield of 52%) expressed by the following formula was obtained as a yellow oil, by decompression-concentrating the product and then refining the concentrate with column chromatography (alumina, hexane) and fractional type GPC (JAIGEL-1H, 2H, eluent CHCl₃).

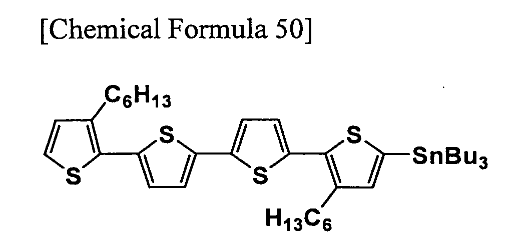

### (Referential example 5 of synthesis)

### <Synthesis of SnBu₃-8T-SnBu₃>

Into a 10 mL two-neck flask of which the inside had been heated, dried and replaced with nitrogen, 8T (4T-4T) (150 mg, 0.15 mmol) was charged, dried THF (5 mL) was added thereto, subsequently, the inside was cooled to -23°C, and tetramethylene diamine (0.09 mL, 0.6 mmol) and 1.6 M n-butyl lithium / hexane (0.38 mL, 0.60 mmol) were added dropwise. After the liquid was stirred for 30 minutes, tributyltin chloride (0.12 mL, 0.66 mmol) was added at a time. The reaction system was heated to room temperature and was stirred for 3 hours. Water (1 mL) and chloroform (20 mL) were added to the reaction solution. After an organic layer was washed twice by using water (20 mL), the product was dried by anhydrous sodium sulfate. A target object (SnBu₃-8T-SnBu₃) (120 mg, yield of 50%) expressed by the following formula was obtained as a red oil, by decompression-concentrating the product and then refining the concentrate with column chromatography (alumina, hexane) and fractional type GPC (JAIGEL-1H, 2H, eluent CHCl₃).

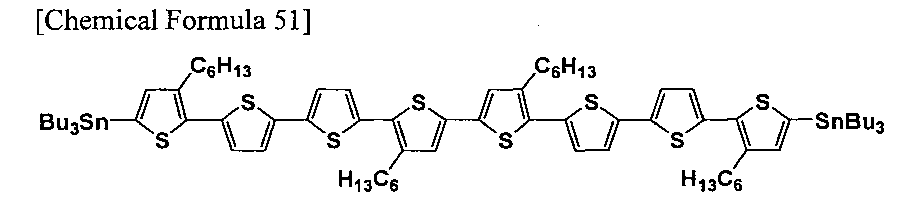

### (Referential example 6 of synthesis)

### <Synthesis of SnBu₃-6T-SnBu₃>

Into a 30 mL two-neck flask of which the inside had been heated, dried and replaced with nitrogen, 5,5"'-dibromo-3,3"'-dihexyl-sexithiophene (418 mg, 0.50 mmol) was charged, dried THF (10 mL) was added thereto, subsequently, the inside was cooled to -78°C, and tetramethylene diamine (0.23 mL, 1.5 mmol) and 1.6 M n-butyl lithium / hexane (0.8 mL, 1.3 mmol) were added dropwise. After the liquid was stirred for 30 minutes, tributyltin chloride (0.38 mL, 1.4 mmol) was added at a time. The reaction system was heated to room temperature and was stirred for 3 hours. Next, water (1 mL) and hexane (20 mL) were added to the reaction solution. After an organic layer was washed twice by using water (20 mL), the product was dried by anhydrous sodium sulfate. A target object (SnBu₃-6T-SnBu₃) (627 mg, 0.44 mmol, yield of 89%) expressed by the following formula was obtained as an orange oil, by decompression-concentrating the product and then refining the concentrate with column chromatography (alumina, hexane).
Orange oil; ¹H-NMR (270 MHz, CDCl₃) δ 0.86-0.94 (m, 30H), 1.11 (t, J=8.1 Hz, 12H), 1.28-1.42 (m, 36H), 1.53-1.70 (m, 20H), 2.74-2.84 (m, 8H), 6.95 (s, 4H), 7.04 (d, J=3.6 Hz, 2H), 7.13 (d, J=3.6 Hz, 2H); ¹³C-NMR (68 MHz, CDCl₃) δ 11.0, 13.8, 14.2, 22.7, 26.9, 27.8, 28.9, 29.0, 29.3, 29.4, 29.6, 30.6, 30.8, 31.8, 123.7, 126.0, 128.1, 129.7, 134.8, 135.1, 135.6, 135.9, 136.5 138.6, 139.8, 140.5; MS (MALDI-TOF, 1, 8, 9-trihydroxyanthracene matrix) m/z 1413.6 (M⁺, Calcd 1408.5); Anal. Calcd for C₇₂H₁₁₄S₆Sn₂: C, 61.35; H, 8.15; Found: C, 61.32; H, 7.94.

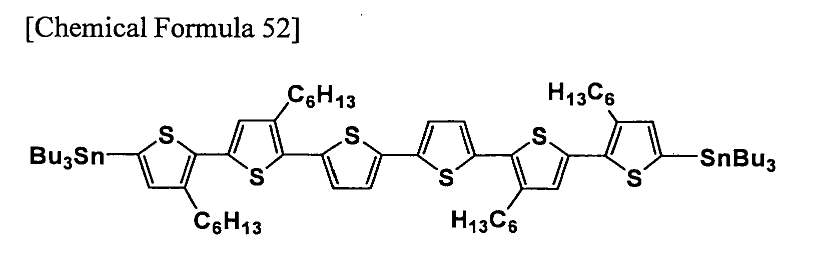

### (Example 1)

### <Synthesis of compound A>

Into a lidded test tube of which the inside had been heated and dried, bis-tributylstannyl-quarter thiophene (SnBu₃-4T-SnBu₃) (2.0 g, 1.86 mmol), N-(1-nonyl decyl)-N'-(4-iodophenyl) perylene diimide (800 mg, 0.93 mmol) and 17 ml of dried toluene were charged. After the liquid was degassed by bubbling, tetrakis (triphenyl phosphine) palladium (0) (54 mg, 0.46 mmol) was added thereto, and the liquid was refluxed under argon atmosphere for 2 hours. A solid material was removed from the product with sellite filtration, and the product was decompression-concentrated. A compound A of a target object (590 mg, yield of 42%) expressed by the following formula was obtained as a red solid, by refining the concentrate with column chromatography (alumina, hexane/chloroform = 5/1 to 1/1). The result of the analysis of the obtained compound A is shown below.
TLC Rf=0.50 (hexane: chloroform); ¹H-NMR (CDCl₃, 400 MHz) δ 0.81-0.93 (m, 21H), 1.10-1.14 (m, 6H), 1.20-1.42 (m, 46H), 1.50-1.61 (m, 6H), 1.60-1.74 (m, 4H), 1.85-1.88 (m, 2H), 2.23-2.27 (m, 2H), 2.80-2.83 (m, 4H), 5.19 (s, 1H), 6.97 (d, J=3.9 Hz, 1H), 7.08 (d, J=3.8 Hz, 1H), 7.14 (d, J=3.9 Hz, 1H), 7.15 (d, J=3.6 Hz, 1H), 7.22 (s, 1H), 7.38 (d, J=8.5 Hz, 2H), 7.78 (d, J=8.5 Hz, 2H), 8.63-8.75 (m, 8H); MS (MALDI-TOF, 1, 8, 9-trihydroxyanthracene matrix) m/z 1524.5 (M⁺, calcd 1518.6); Anal. Calcd for C₁₆₀H₁₆₉BrN₄O₈S₈: C, 70.38; H, 7.30; N, 1.84; Found: C, 70.10; H, 7.33; N, 1.69.

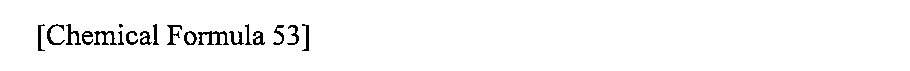

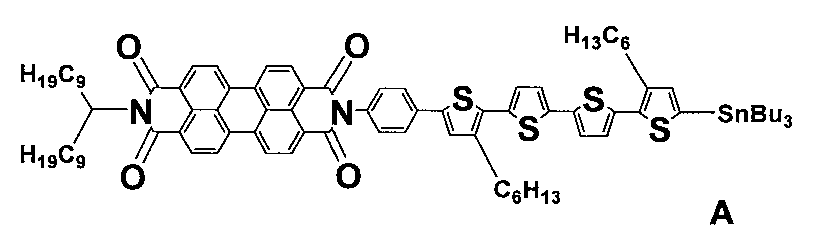

### <Synthesis of compound B>

Into a lidded test tube of which the inside had been heated and dried, the compound A (760 mg, 0.5 mmol), 3,5-diiodobromobenzene (89 mg, 0.22 mmol) and dried toluene (7 mL) were charged. After the liquid was degassed by bubbling, tetrakis (triphenyl phosphine) palladium (0) (19 mg, 0.0165 mmol) was added thereto, and the liquid was refluxed under argon atmosphere for 12 hours. A solid material was removed from the product with sellite filtration, and the product was decompression-concentrated. A compound B of a target object (450 mg, yield of 78%) expressed by the following formula was obtained as a dark red solid, by refining the concentrate with column chromatography (silica gel, chloroform) and fractional type GPC (JAIGEL-1H, 2H, eluent chloroform). The result of the analysis of the obtained compound B is shown below.
TLC R_{f}=0.20 (chloroform); ¹H-NMR (CDCl₃, 400 MHz) δ 0.81-0.85 (m, 12H), 0.91-0.94 (m, 12H), 1.20-1.44 (m, 80H), 1.71 (m, 8H), 1.89 (m, 4H), 2.25 (m, 4H), 2.81 (m, 8H), 5.18 (m, 2H), 7.09 (d, J=3.9 Hz, 4H), 7.16 (d, J=3.9 Hz, 2H), 7.17 (d, J=3.9 Hz, 2H), 7.17 (s, 2H), 7.21 (s, 2H), 7.36 (d, J=8.6 Hz, 4H), 7.57 (m, 3H), 7.76 (d, J=8.5 Hz, 4H), 8.58-8.72 (m, 16H); MS (MALDI-TOF, 1, 8, 9-trihydroxyanthracene matrix) m/z 2608.7 (M⁺, calcd 2612.0); Anal. Calcd for C₁₆₀H₁₆₉BrN₄O₈S₈: C, 73.56; H, 6.52; N, 2.14.Found: C, 73.30; H, 6.52; N, 2.01.

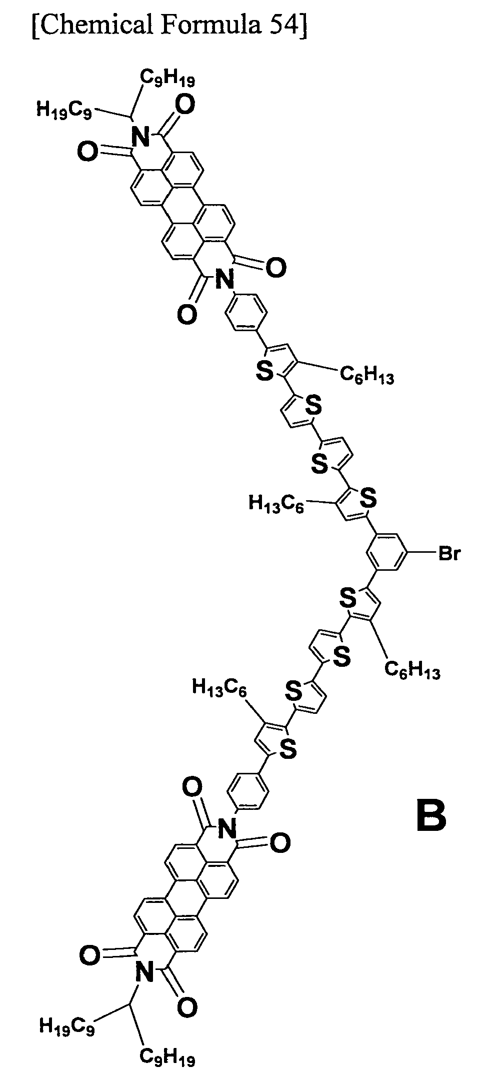

### <Synthesis of compound C>

Into a lidded test tube of which the inside had been heated and dried, the compound B (292 mg, 0.112mmol), bis-tributylstannyl-quarter thiophene (SnBu₃-4T-SnBu₃) (55 mg, 0.51 mmol) and dried toluene (1.2 mL) were charged. After the liquid was degassed by bubbling, tetrakis (triphenyl phosphine) palladium (0) (5.0 mg, 5.1 µmol) was added thereto, and the liquid was refluxed under argon atmosphere for 8 hours. A solid material was removed from the product with sellite filtration, and the product was decompression-concentrated. A compound C of a target object (93 mg, yield of 47%) expressed by the following formula was obtained as a dark red solid, by refining the concentrate with column chromatography (alumina, chloroform) and fractional type GPC (JAIGEL-3H, 4H, eluent chloroform). The result of the analysis of the obtained compound C is shown below.
TLC R_{f}=0.3 (chloroform); ¹H-NMR (CDCl₃, 400 MHz) δ 0.81-0.91 (m, 54H), 1.20-1.33 (m, 172H), 1.69 (m, 20H), 1.89 (m, 8H), 2.22 (m, 8H), 2.77 (m, 20H), 5.16 (m, 4H), 6.96-7.22 (m, 30H), 7.30 (m, 8H), 7.52-7.61 (m, 6H), 7.73 (m, 8H), 8.24-8.70 (m, 32H); MS (MALDI-TOF, 1, 8, 9-trihydroxyanthracene matrix) m/z 5569 (M⁺, calcd 5559). UV-Vis spectra in CHCl₃, absₘₐₓ=430 nm, 460 nm, 490 nm, 530 nm.

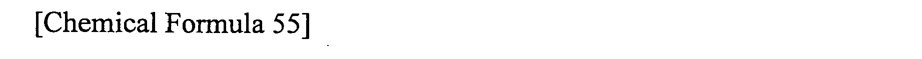

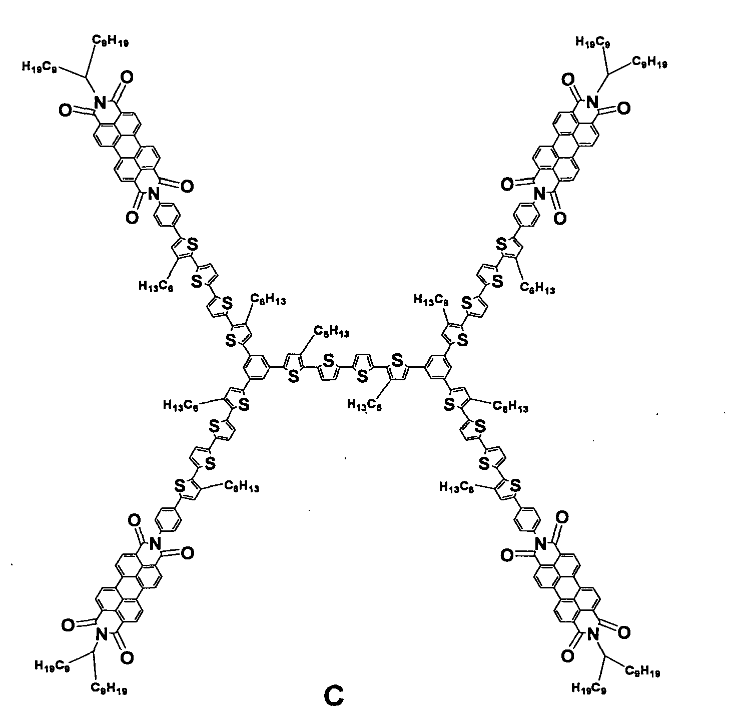

As a result of cyclic voltammetry (CV) measurement, it could be confirmed that the compound C acquired an oxidation potential (E_{p.a}) of +0.48 and a reduction potential (Ered) of -1.11 and -1.31, and could show ambipolar conduction.

### (Example 2)

### <Synthesis of compound D>

Into a lidded test tube of which the inside had been heated and dried, the compound B (500 mg, 0.19 mmol), bis-tributyl stannyl-octathiophene (SnBu₃-8T-SnBu₃) (120 mg, 0.076 mmol) and dried toluene (3 mL) were charged. After the liquid was degassed by bubbling, tetrakis (triphenyl phosphine) palladium (0) (9 mg, 7.6 µmol) was added thereto, and the liquid was refluxed under argon atmosphere for 5 hours. A solid material was removed from the product with sellite filtration, and the product was decompression-concentrated. A compound D of a target object (200 mg, yield of 43%) expressed by the following formula was obtained as a dark red solid, by refining the concentrate with column chromatography (alumina, chloroform) and fractional type GPC (JAIGEL-3H, 4H, eluent chloroform). The result of the analysis of the obtained compound D is shown below.
TLC R_{f}=0.3 (chloroform); ¹H-NMR (CDCl₃, 400 MHz) δ 0.81-0.91 (m, 54H), 1.20-1.33 (m, 172H), 1.69 (m, 20H), 1.89 (m, 8H), 2.22 (m, 8H), 2.77 (m, 20H), 5.16 (m, 4H), 6.96-7.22 (m, 30H), 7.30 (m, 8H), 7.52-7.61 (m, 6H), 7.73 (m, 8H), 8.24-8.70 (m, 32H); MS (MALDI-TOF, 1, 8, 9-trihydroxyanthracene matrix) m/z 6055.0 (M⁺, calcd 6058.4). UV-Vis spectra in CHCl₃, absₘₐₓ=430 nm, 460 nm, 490 nm, 530 nm. Anal. Calcd for C₃₇₆H₄₀₂N₈O₁₆S₂₄: C, 74.54; H, 6.69; N, 1.85. Found: C, 74.30; H, 6.45; N, 1.74.

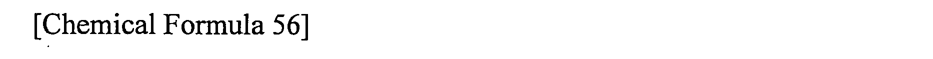

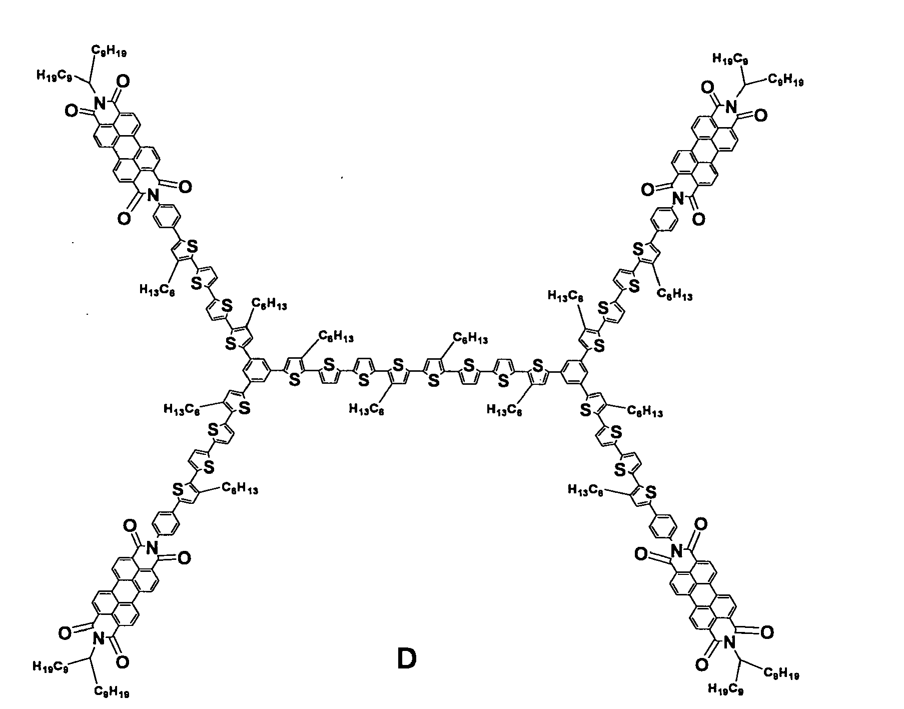

As a result of cyclic voltammetry (CV) measurement, it could be confirmed that the compound D acquired an oxidation potential (E_{p.a.}) of +0.45 and a reduction potential (E_{red}) of -1.14 and -1.39, and could show ambipolar conduction.

### (Comparative example 1)

### <Synthesis of compound E>

Nitrogen gas was blown into an anhydrous toluene solution (50 mL) containing 1,3,5-tribromobenzene (89 mg, 0.28 mmol) and 4T-SnBu₃ (445 mg, 0.57 mmol) for 15 minutes under a stream of nitrogen to degas the toluene solution. Then, Pd(PPh₃)₄ (33 mg, 28 µmol) was added to the solution, and the liquid was refluxed for 12 hours. Then, a black precipitation was removed with sellite filtration, and the solvent was distilled off under a reduced pressure. A compound E (122 mg, yield of 38 %, orange film) expressed by the following formula was isolated from the obtained mixture with column chromatography (silica gel, methylene chloride: hexane / 1:9, R_{f}= 0.3).
¹H-NMR (CDCl₃, 270 MHz) δ 0.86-0.95 (m, 12H), 1.26-1.46 (m, 24H), 1.63-1.78 (m, 8H), 2.76-2.84 (m, 8H), 6.94 (d, J=5.1 Hz, 2H), 7.03 (d, J=3.8 Hz, 2H), 7.09 (d, J=3.5 Hz, 2H), 7.15 (d, J=3.8 Hz, 2H), 7.15 (d, J=3.5 Hz, 2H), 7.18 (d, J=5.1 Hz, 2H), 7.21 (s, 2H), 7.62 (d, J=3.5 Hz, 2H), 7.66 (t, J=1.6 Hz, 1H).

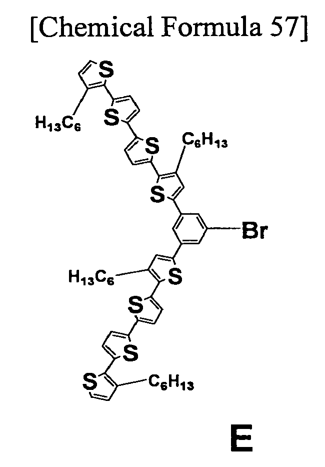

### <Synthesis of compound F>

Nitrogen gas was blown into an anhydrous toluene solution (20 mL) containing SnBu₃-4T-SnBu₃ (52 mg, 48 µmol) and the compound E (122 mg, 0.11 mmol) for 15 minutes under a stream of nitrogen to degas the toluene solution. Then, Pd(PPh₃)₄ (6 mg, 5 µmol) was added to the solution, and the liquid was refluxed for 12 hours. Then, a black precipitation was removed with sellite filtration, and the solvent was distilled off under a reduced pressure. A compound F (54 mg, yield of 43 %, red film) expressed by the following formula was isolated from the obtained mixture with column chromatography (silica gel, methylene chloride: hexane / 1:3, R_{f} = 0.2).
¹H-NMR (CDCl₃, 270 MHz) δ 0.86-0.95 (m, 30H), 1.25-1.49 (m, 60H), 1.66-1.75 (m, 20H), 2.76-2.87 (m, 20H), 6.95 (d, J=5.4 Hz, 4H), 7.04 (d, J=3.8 Hz, 4H), 7.11 (d, J=3.8 Hz, 4H), 7.12 (d, J=3.8 Hz, 2H), 7.15 (d, J=3.8 Hz, 4H), 7.16 (d, J=3.8 Hz, 4H), 7.18 (d, J=5.4, 4H), 7.19 (d, J=3.8 Hz, 2H), 7.28 (s, 6H), 7.68 (s, 6H).

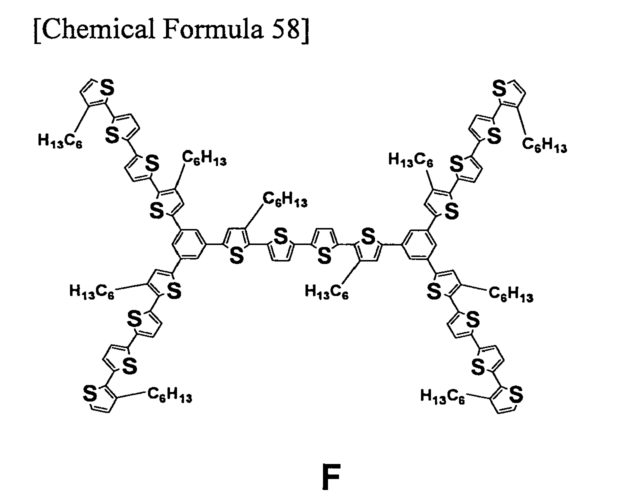

As a result of CV measurement, the compound F could obtain a peak (E_{p.a.} = +0.30) in an oxidization side, but showed inadequate and unstable cyclability. The compound F could not obtain a peak in a reduction side, and did not show ambipolar conduction.

### (Example 3)

### <Synthesis of compound G>

Into a 50 mL two-neck flask of which the inside had been heated, dried and replaced with nitrogen, quarter thiophene-carboxyaldehyde (4T-CHO) was charged, dried THF (14 mL) was added thereto, subsequently, the inside was cooled to -78°C, and n-butyl lithium / hexane was added dropwise. After the liquid was stirred for 30 minutes, tributyltin chloride was added at a time. The reaction system was heated to room temperature and was stirred for 3 hours. Water and hexane were added to the reaction solution, an organic layer was washed twice with water, and the product was dried by anhydrous magnesium sulfate. Tributylstannyl-quarter thiophene-carboxyaldehyde (SnBu₃-4T-CHO) of a target object was obtained by removing an insoluble matter by filtration, decompression-concentrating the product and refining the concentrate with column chromatography (alumina, hexane/dichloromethane = 9/1).

Into a two-neck flask of which the inside had been replaced with nitrogen, tributylstannyl-quarter thiophene (4T-SnBu₃) and 3,5-dibromoiodobenzene were charged, and dried toluene was added thereto. After the liquid was degassed by bubbling, tetrakistriphenyl phosphine palladium (0) was added thereto, and the liquid was heated and refluxed for 12 hours. PhBr₂-4T of a target object was obtained as a yellow solid by removing a solid material with sellite filtration, decompression-concentrating the product, and refining the concentrate with column chromatography (silica gel, hexane) and recrystallization (hexane).

Into a two-neck flask of which the inside had been replaced with nitrogen, SuBu₃-4T-CHO and PhBr₂-4T were charged, and dried toluene was added thereto. After the liquid was degassed by bubbling, tetrakistriphenyl phosphine palladium (0) was added thereto, and the liquid was heated and refluxed for 12 hours. A compound G (G1(CHO)-4T) of a target object expressed by the following formula was obtained by removing a solid material with sellite filtration, decompression-concentrating the product, and refining the concentrate with column chromatography (silica gel, hexane/dichloromethane = 9/1) and recrystallization (hexane/dichloromethane).

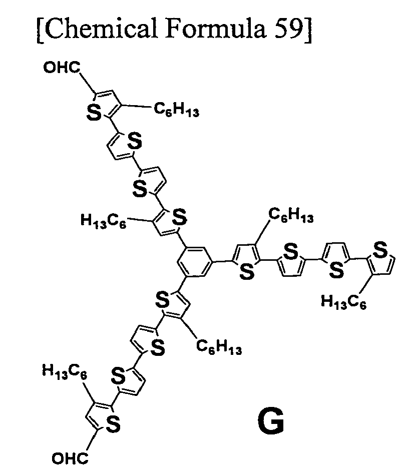

### <Synthesis of compound H>

Into a 20 mL two-neck flask of which the inside had been heated, dried and replaced with nitrogen, the compound G was charged, dried THF was added thereto, subsequently, the inside was cooled to - 78°C, and 1.6 M n-butyl lithium / hexane was added dropwise. After the liquid was stirred for 30 minutes, tributyltin chloride was added at a time. The reaction system was heated to room temperature and was stirred for 3 hours, water and chloroform were added to the reaction solution, an organic layer was washed twice with water, and the product was dried by anhydrous magnesium sulfate. A compound H (G1(CHO)-4T-SnBu₃) of a target object expressed by the following formula was obtained by removing an insoluble matter by filtration, decompression-concentrating the product and refining the concentrate with column chromatography (alumina, hexane/dichloromethane = 4/1).

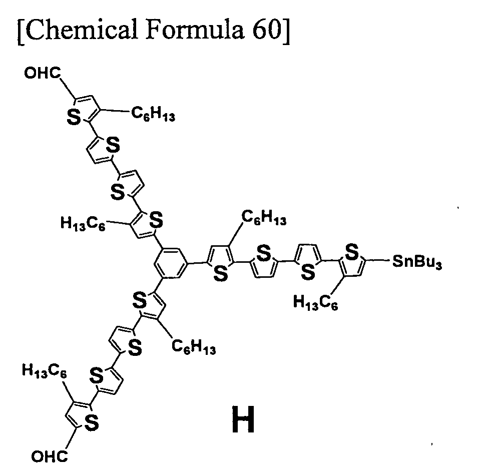

### <Synthesis of compound I>

Into a lidded test tube of which the inside had been heated and dried, the compound H (G1(CHO)-4T-SnBu₃)_{,} 3,5-diiodobromobenzene and dried toluene were charged. After the liquid was degassed by bubbling, tetrakis (triphenyl phosphine) palladium (0) was added thereto, and the liquid was refluxed under argon atmosphere. A compound I of a target object expressed by the following formula was obtained by removing a solid material with sellite filtration, decompression-concentrating the product, and refining the concentrate with column chromatography and fractional type GPC.

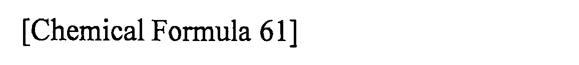

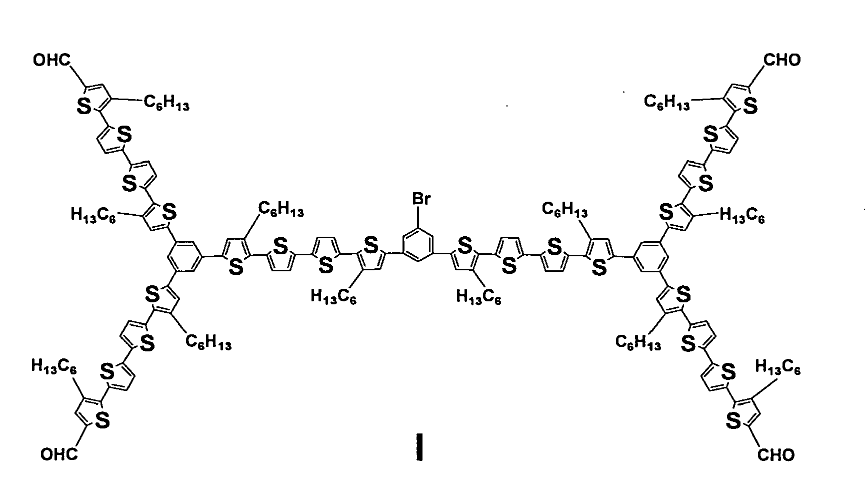

### <Synthesis of compound J>

Into a two-neck flask of which the inside had been replaced with nitrogen, the compound I and Ph-8T-SnBu₃ were charged, and dried toluene was added thereto. After the liquid was degassed by bubbling, tetrakistriphenyl phosphine palladium (0) was added thereto, and the liquid was heated and refluxed. A compound J of a target object expressed by the following formula was obtained by removing a solid material with sellite filtration, decompression-concentrating the product, and refining the concentrate with column chromatography (silica gel, hexane/dichloromethane = 9/1) and recrystallization (hexane/dichloromethane).

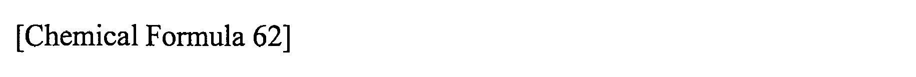

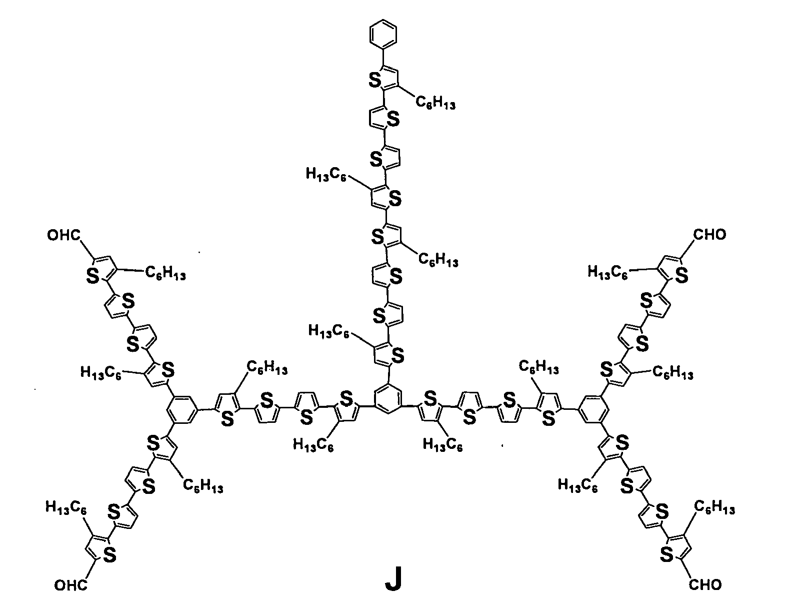

### <Synthesis of compound K>

Into a lidded test tube of which the inside had been heated and dried, the compound J, C₆₀, N-methyl glycine and dried toluene were charged. After the liquid was degassed by bubbling, tetrakis (triphenyl phosphine) palladium (0) was added thereto, the liquid was refluxed under argon atmosphere, a solid material was removed from the product with sellite filtration, and the product was decompression-concentrated. A compound K of a target object expressed by the following formula was obtained by refining the concentrate with column chromatography and fractional type GPC.

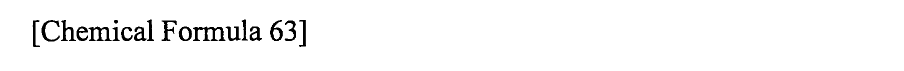

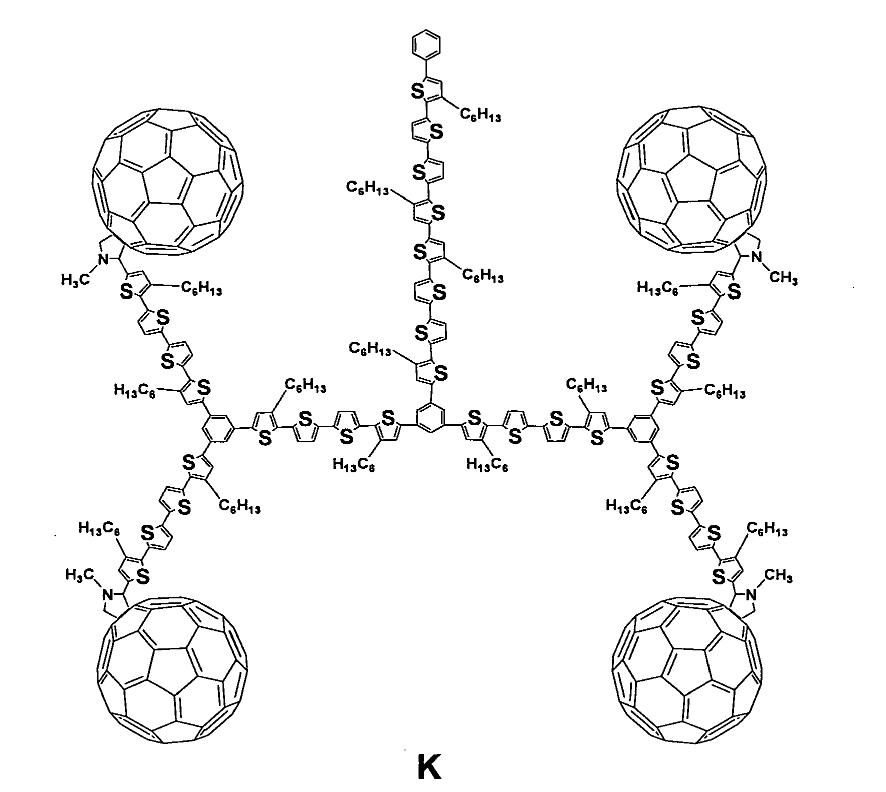

### (Example 4)

### <Synthesis of compound L>

Into a lidded test tube of which the inside had been heated and dried, (SnBu₃-4T-CHO), 3,5-diiodobromobenzene and dried toluene were charged. After the liquid was degassed by bubbling, tetrakis (triphenyl phosphine) palladium (0) was added thereto, and the liquid was refluxed under argon atmosphere. A solid material was removed from the product with sellite filtration, and the product was decompression-concentrated. A compound L of a target object expressed by the following formula was obtained by refining the concentrate with column chromatography and fractional type GPC.

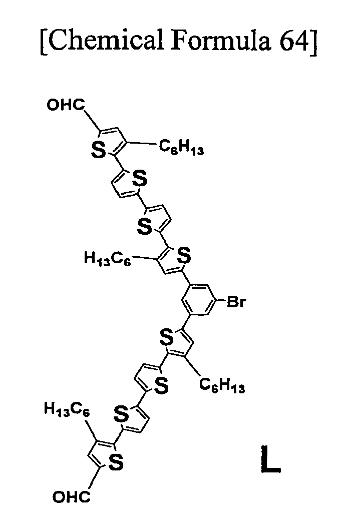

### <Synthesis of compound M>

Into a lidded test tube of which the inside had been heated and dried, the compound L, SnBu₃-8T-SnBu and dried toluene were charged. After the liquid was degassed by bubbling, tetrakis (triphenyl phosphine) palladium (0) was added thereto, and the liquid was refluxed under argon atmosphere. A solid material was removed from the product with sellite filtration, and the product was decompression-concentrated. A compound M of a target object expressed by the following formula was obtained by refining the concentrate with column chromatography and fractional type GPC.

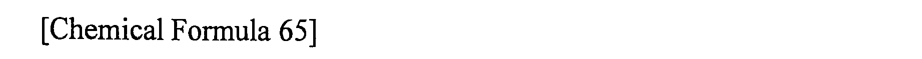

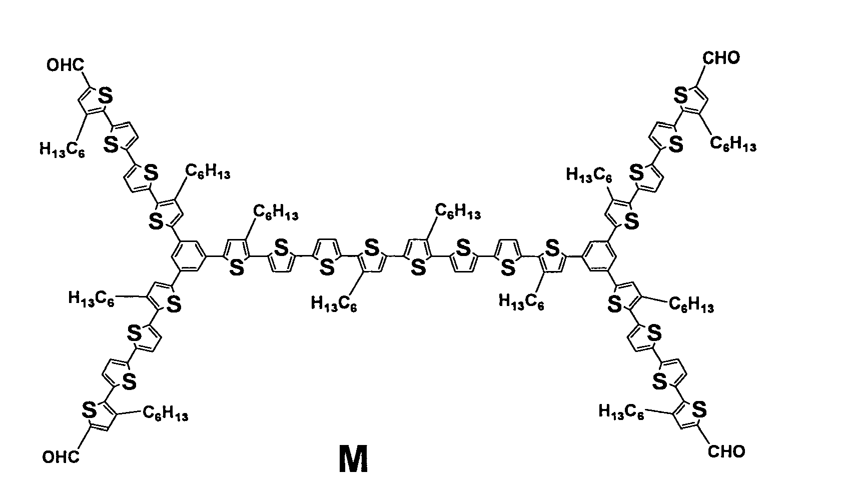

### <Synthesis of compound N>

Into a lidded test tube of which the inside had been heated and dried, the compound M, C₆₀, N-methyl glycine and dried toluene were charged. After the liquid was degassed by bubbling, tetrakis (triphenyl phosphine) palladium (0) was added thereto, and the liquid was refluxed under argon atmosphere. A solid material was removed from the product with sellite filtration, and the product was decompression-concentrated. A compound N of a target object expressed by the following formula was obtained by refining the concentrate with column chromatography and fractional type GPC.

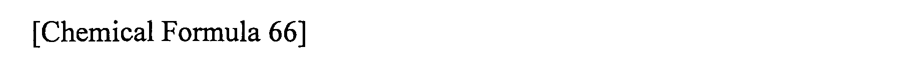

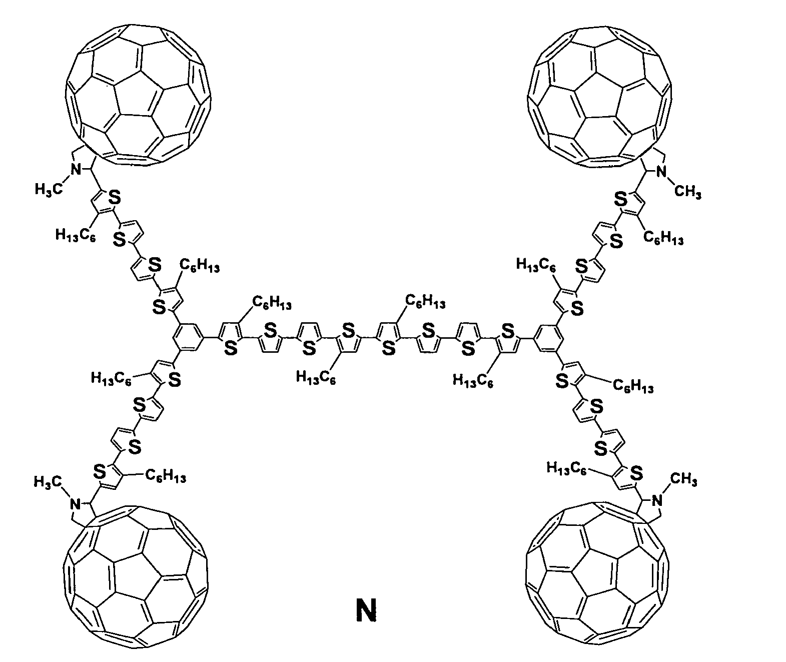

### (Example 5)

### <Production of organic thin film device 1, and evaluation of solar cell characteristics>

On a glass substrate on which ITO had been deposited to be the thickness of 150 nm, an organic thin film (with the thickness of 100 nm) of the compound C which was synthesized in Example 1 was formed with a spin coating method by using a 1.0 mass% chloroform solution of the compound C. Next, on the formed organic thin film, an Al electrode was vacuum-deposited to be the thickness of 100 nm, and the organic thin film device 1 was produced.

The organic thin film device 1 was irradiated with the light of 10 µW/cm², which had a wavelength of 470 nm dispersed from a xenon lamp, and thereby, solar cell characteristics in the wavelength of 470 nm were measured. A short-circuit current of 0.204 µA/cm², an open voltage of 0.46 V, a spectral responsivity of IPCE = 5.5% and a photoelectric conversion efficiency of 0.25 were obtained; and it was confirmed that the organic thin film device 1 functioned as the organic solar cell.

After the organic thin film device 1 was annealed in nitrogen atmosphere at 120°C for 10 minutes, the solar cell characteristics were measured under the same conditions. The short-circuit current of 0.321 µA/cm², the open voltage of 0.81 V, the spectral responsivity of IPCE = 8.5% and the photoelectric conversion efficiency of 0.66 were obtained, and the solar cell characteristics were improved.

### (Example 6)

### <Production of organic thin film device 2, and evaluation of solar cell characteristics>

On a glass substrate on which an aluminum electrode had been deposited to be the thickness of 10 nm, an organic thin film (with the thickness of 100 nm) of the compound K which was synthesized in Example 3 was formed with a spin coating method by using a 1.0 mass% chloroform solution of the compound K. Next, on the formed organic thin film, a gold electrode was vacuum-deposited to be the thickness of 10 nm, and the organic thin film device 2 was produced.

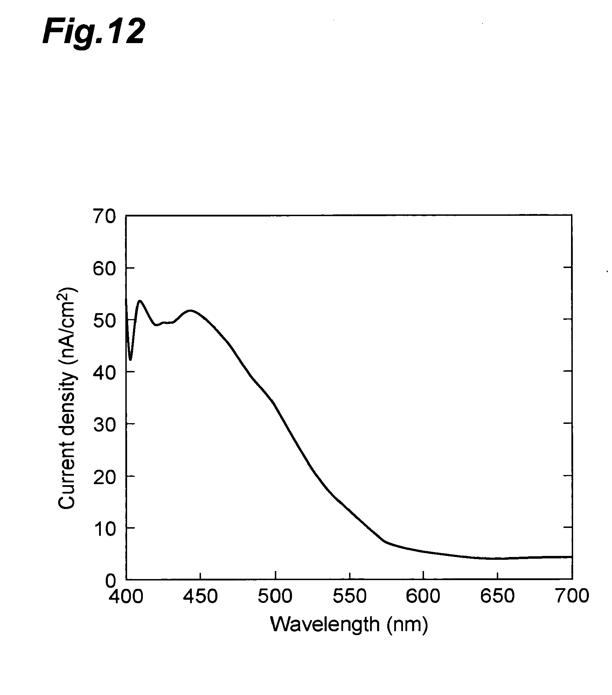

The organic thin film device 2 was irradiated with the light of 10 µW/cm², and spectral responsivity at this time was measured. As shown in Figure 12, adequate sensitivity was obtained in a wavelength range of 400 to 550 nm, and it was confirmed that the organic thin film device 2 functioned as the organic solar cell and a light sensor.

### (Example 7)

### <Production of organic thin film device 3, and evaluation of solar cell characteristics>

The organic thin film device 3 was produced in a similar way to that in Example 5 except that the compound D which was synthesized in Example 2 was used in place of the compound C.

The organic thin film device 3 was irradiated with the light of 10 µW/cm², which had a wavelength of 410 nm dispersed from a xenon lamp, and thereby, solar cell characteristics in the wavelength of 410 nm were measured. A short-circuit current of 0.224 µA/cm², an open voltage of 0.66 V, a spectral responsivity of IPCE = 6.7% and a photoelectric conversion efficiency of 0.4 were obtained; and it was confirmed that the organic thin film device 3 functioned as the organic solar cell.

### (Example 8)

### <Synthesis of compound O>

Into a lidded test tube of which the inside had been heated and dried, 2, 2, 3, 3, 4, 4, 4-heptafluorobutylamine (1.00 g, 5.03 mmol), 4-iodine aniline (1.10 g, 5.03 mmol), naphthalene tetracarboxydianhydride (1.35 g, 5.03 mmol), acetic acid (2.4 g, 0.4 mmol) and 25 ml of N-methylpyrrolidone (NMP) were charged. After the liquid was degassed by bubbling, the liquid was made to react at 90°C under N₂ atmosphere all night. Ethanol was added to the reaction solution, a precipitated solid material was removed from the product with sellite filtration, and the product was decompression-concentrated. Subsequently, water was added, and the precipitated solid material was taken through sellite filtration. A compound O of a target object (780 mg, yield of 25%) expressed by the following formula was obtained as a yellow solid, by recrystallization-refining the obtained solid material with acetone, and further refining the material with column chromatography (alumina, hexane/chloroform = 1/1). The result of the analysis of the obtained compound O is shown below.
TLC Rf=0.3 (hexane: CHCl₃=1: 1); ¹H-NMR (CDCl₃, 400 MHz) δ 5.04 (t, J=15.4 Hz, 2H), 7.08 (d, J=6.5 Hz, 2H), 7.91 (d, J=6.5 Hz, 1H), 8.84-8.88 (m, 4H): MS (MALDI-TOF, 1, 8, 9-trihydroxyanthracene matrix) m/z 650.3 (M⁺, calcd 649.0)

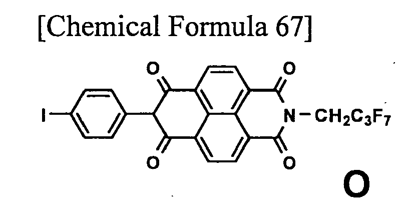

### <Synthesis of compound P>

Into a lidded test tube of which the inside had been heated and dried, SnBu₃-6T-SnBu₃ (1.60 g, 1.13 mmol), the compound O (300 mg, 0.462 mmol) and 15 ml of dried toluene were charged. After the liquid was degassed by bubbling, tetrakis (triphenyl phosphine) palladium (0) (25 mg, 0.023 mmol) was added thereto, and the liquid was refluxed under N₂ atmosphere for 2 hours. A compound P of a target object (180 mg, yield of 24%) expressed by the following formula was obtained as a red solid, by decompression-concentrating the product, and then refining the concentrate with column chromatography (alumina, hexane/chloroform = 2/1). The result of the analysis of the obtained compound P is shown below.
TLC Rf=0.2 (CHCl₃); ¹H-NMR (CDCl₃, 400 MHz) δ 0.90-0.93 (m, 21H), 1.09-1.13 (m, 6H), 1.32-1.38 (m, 30H), 1.57-1.71 (m, 14H), 2.78-2.84 (m, 8H), 5.00-5.08 (m, 2H), 6.95-7.14 (m, 8H), 7.33-7.35 (m, 2H), 7.77-7.79 (m, 2H), 8.83-8.88 (m, 4H): MS (MALDI-TOF, 1, 8, 9-trihydroxy-anthracene matrix) m/z 1642.4 (M⁺, calcd 1641.5)

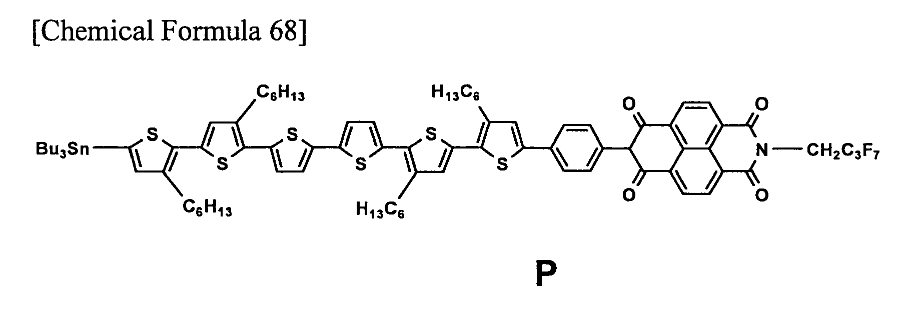

### <Synthesis of compound Q>

Into a lidded test tube of which the inside had been heated and dried, the compound P (180 mg, 0.112 mmol), 3,5-diiodobromobenzene (22 mg, 0.055 mmol) and 4 mL of dried toluene were charged. After the liquid was degassed by bubbling, tetrakis (triphenyl phosphine) palladium (0) (4 mg, 4 µmol) was added thereto, and the liquid was refluxed under N₂ atmosphere for 3 hours. A compound Q of a target object (60 mg, yield of 37%) expressed by the following formula was obtained as a dark red solid, by decompression-concentrating the product and then refining the concentrate with column chromatography (silica gel, chloroform) and fractional type GPC (JAIGEL-1H, 2H, eluent CHCl₃). The result of the analysis of the obtained compound Q is shown below.
TLC Rf=0.2 (CHCl₃); ¹H-NMR (CDCl₃, 400 MHz) δ 0.91-0.93 (m, 24H), 1.34-1.46 (m, 48H), 1.69-1.74 (m, 16H), 2.79-2.83 (m, 16H), 5.00-5.08 (m, 4H), 7.03-7.23 (m, 16H), 7.33-7.35 (m, 4H), 7.61-7.66 (m, 3H), 7.77-7.79 (m, 4H), 8.85-8.89 (m, 8H): MS (MALDI-TOF, 1, 8, 9-trihydroxy-anthracene matrix) m/z 2858.66 (M⁺, calcd 2903.7)

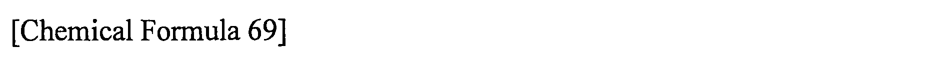

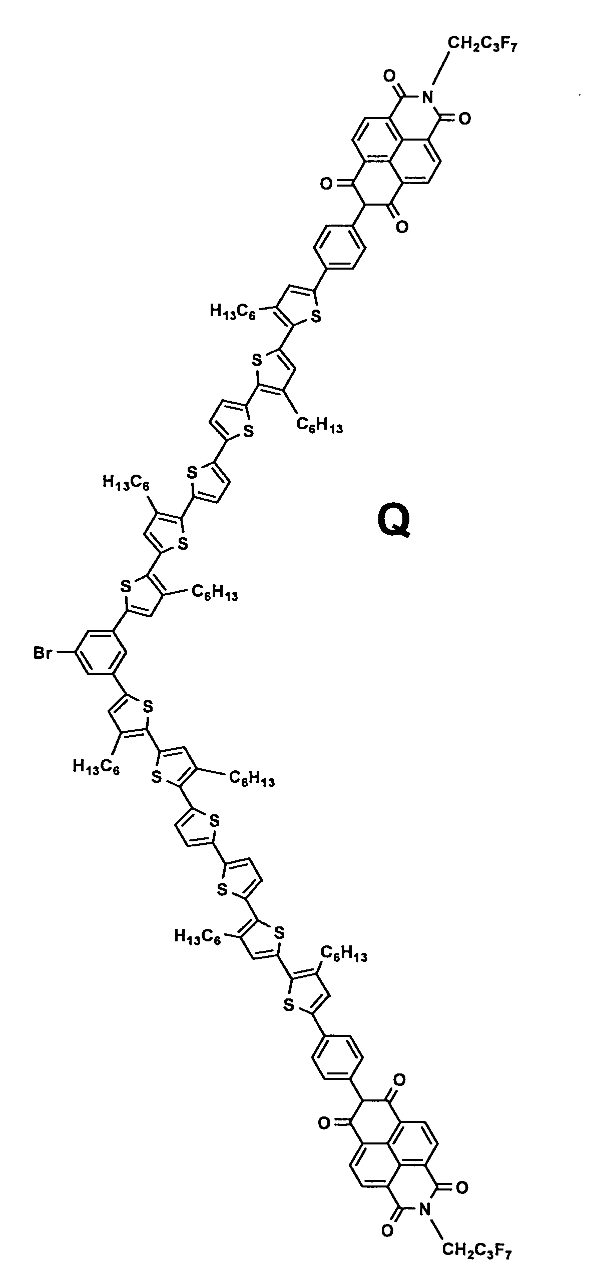

### <Synthesis of compound R>

Into a lidded test tube of which the inside had been heated and dried, the compound Q (60 mg, 0.021 mmol), SnBu₃-6T-SnBu₃ (15 mg, 0.01 mmol) and 3.0 ml of dried toluene were charged. After the liquid was degassed by bubbling, tetrakis (triphenyl phosphine) palladium (0) (1.2 mg, 1.0 µmol) was added thereto, and the liquid was refluxed under N₂ atmosphere for 5 hours. A compound R of a target object (20 mg, yield of 32%) expressed by the following formula was obtained as a red solid, by decompression-concentrating the product and then refining the concentrate with column chromatography (silica gel, chloroform) and fractional type GPC (JAIGEL-3H, 4H, eluent CHCl₃). The result of the analysis of the obtained compound R is shown below.
TLC Rf=0.2 (CHCl₃); ¹H-NMR (CDCl₃, 400 MHz) δ □0.90-0.95 (m, 60H), 1.26-1.51 (m, 120H), 1.70-1.75 (m, 40H), 2.79-2.83 (m, 40H), 4.98-5.05 (m, 8H), 7.00-7.24 (m, 40H), 7.30-7.32 (d, 8H), 7.62 (s, 6H), 7.75-7.77 (m, 8H), 8.82-8.86 (m, 16H): MS (MALDI-TOF, 1, 8, 9-trihydroxy-anthracene matrix) m/z 6357.52 (M⁺, calcd 6384.79)
UV-Vis spectra in CHCl₃, absₘₐₓ=360 nm, 380 nm, 430 nm

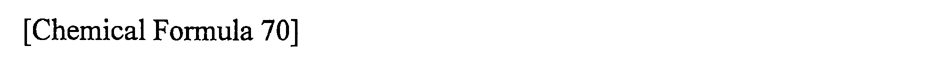

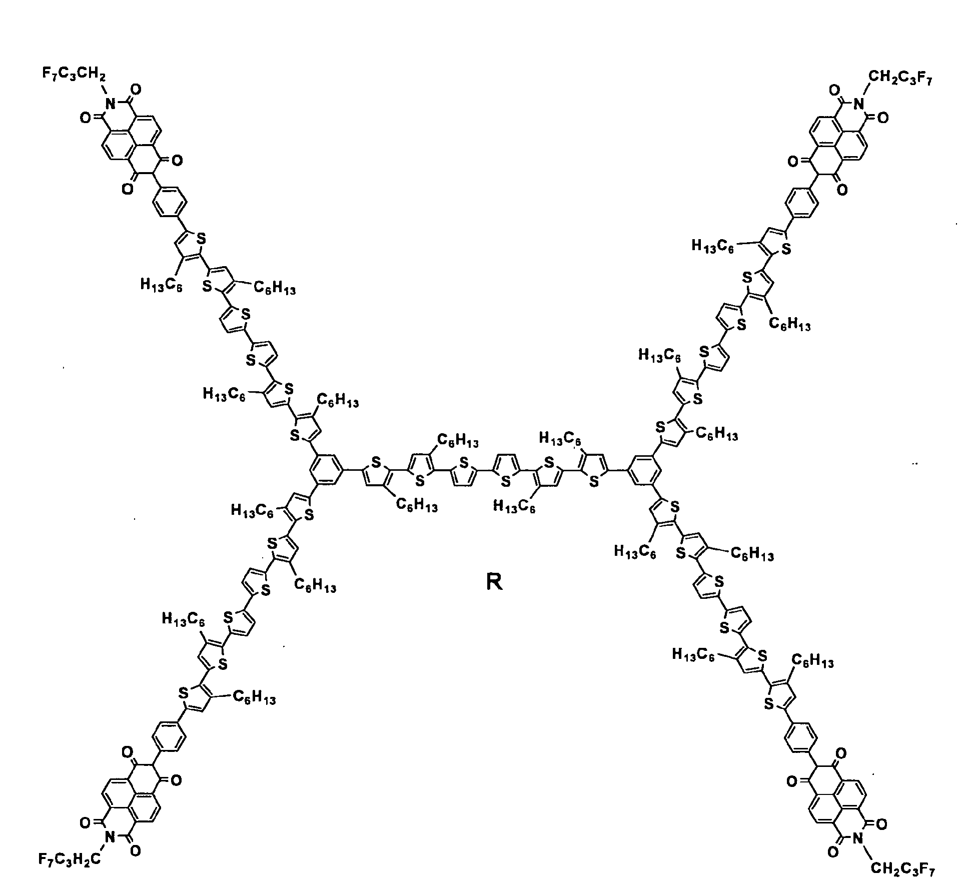

As a result of cyclic voltammetry (CV) measurement, it could be confirmed that the compound R acquired an oxidation potential (E_{p.a}) of +0.26, and a reduction potential (E_{red}) of -1.05 and -1.51, and could show ambipolar conduction.

### (Comparative example 2)

### <Synthesis of compound S>

Into a lidded test tube of which the inside had been heated and dried, the compound A (200 mg, 0.13 mmol), copper (II) chloride (36 mg, 0.26 mmol) and dried toluene were charged. After the liquid was degassed by bubbling, palladium acetate (II) (3.00 mg, 0.013 mmol) was added thereto, and the liquid was made to react under argon atmosphere at room temperature for 15 minutes. A solid material was removed from the product with sellite filtration, and the product was decompression-concentrated. A compound S of a target object (50 mg, yield of 30%) expressed by the following formula was obtained as a dark red solid, by refining the concentrate with column chromatography (silica gel, chloroform) and fractional type GPC (JAIGEL-1H, 2H, eluent CHCl₃). The result of the analysis of the obtained compound S is shown below.
Dark-red solid; Mp207-209°C; TLC Rf=0.5 (CHCl₃); ¹H-NMR (CDCl₃) δ 0.81-0.85 (m, 12H), 0.90-0.94 (m, 12H), 1.21-1.53 (m, 80H), 1.67-1.71 (m, 8H), 1.85-1.91 (m, 4H), 2.21-2.29 (m, 4H), 2.74-2.83 (m, 8H), 5.15-5.22 (m, 2H), 6.99 (s, 2H), 7.04 (d, J=3.9 Hz, 2H), 7.08 (d, J=3.9 Hz, 2H), 7.14 (d, 4H), 7.22 (s, 2H), 7.36 (d, J=8.5 Hz, 4H), 7.76 (d, J=8.5 Hz, 4H), 8.61-8.74 (m, 16H); MS (MALDI-TOF, 1, 8, 9-trihydroxyanthracene matrix) m/z 2458.7 (M⁺, calcd 2456.1); Anal. Calcd for C₁₅₄H₁₆₆N₄O₈S₈: C, 75.27; H, 6.81; N, 2.28; Found: C, 74.89; H, 6.60; N, 2.15
UV-Vis spectra in CHCl₃, absₘₐₓ=440 nm, 460 nm, 490 nm, 530 nm

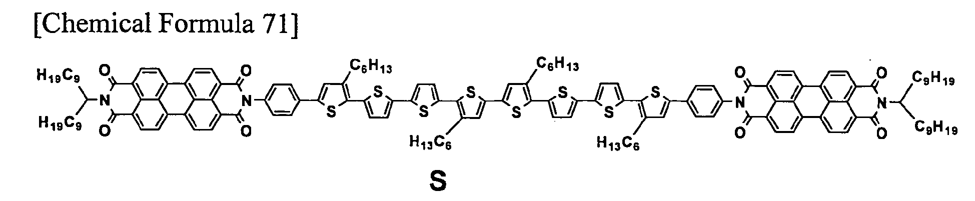

As a result of cyclic voltammetry (CV) measurement, the compound S acquired an oxidation potential (E_{p.a}) of +0.25, and a reduction potential (E_{red}) of -1.11 and -1.32, and could show ambipolar conduction.

### (Comparative example 3)

### <Production of organic thin film device 4, and evaluation of solar cell characteristics>

The organic thin film device 4 was produced in a similar way to that in Example 5 except that the compound S which was synthesized in Comparative example 2 was used in place of the compound C.

The organic thin film device 4 was irradiated with the light of 10 µW/cm², which had a wavelength of 460 nm dispersed from a xenon lamp, and thereby, solar cell characteristics in the wavelength of 460 nm were measured. A short-circuit current of 0.025 µA/cm², an open voltage of 0.22 V, a spectral responsivity of IPCE = 0.67% and a photoelectric conversion efficiency of 0.014 were obtained; and it was confirmed that the organic thin film device 4 functioned as the organic solar cell, but the characteristics are remarkably low.

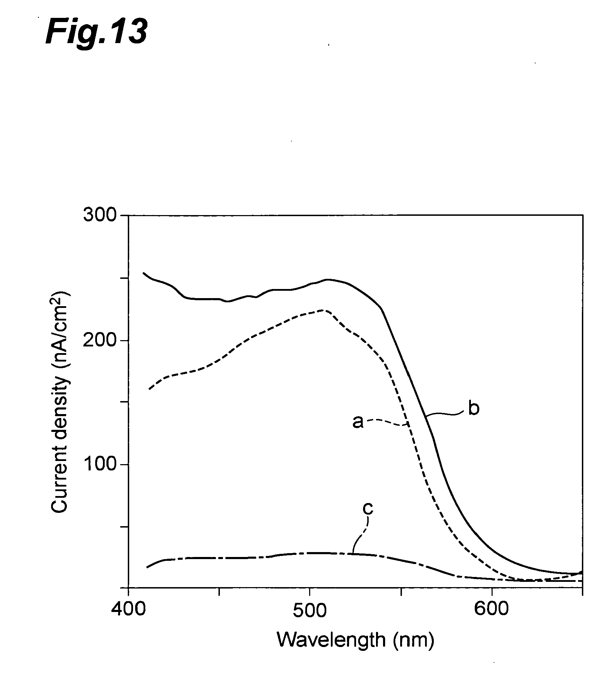

For the purpose of comparison, spectral responsivity characteristics of the organic thin film devices 1, 3, and 4 obtained above is shown in Figure 13. In Figure 13, a graph a shows spectral responsivity characteristics of the organic thin film device 1, a graph b shows spectral responsivity characteristics of the organic thin film device 3, and a graph c shows spectral responsivity characteristics of the organic thin film device 4, respectively.

### Industrial Applicability

The present invention can provide a new branched compound which can be used as a bipolar organic semiconductor excellent in electric-charge transportability. In addition, the present invention can provide an organic thin film containing this branched compound, and an organic thin film device having this organic thin film. In addition, this organic thin film device can be excellent in stability.

## Claims

1. A branched compound comprising a core part, at least one side chain part bonded to the core part, and an end, wherein
one repeating unit or two or more repeating units expressed by the following formula (1) repeat in the or each side chain part, with the proviso that in a repeating unit bonded to the core part, T is bonded to the core part, and in two or more contiguous repeating units, each L is bonded to T;
each L is formed of a plurality of conjugation-forming units linked together;
each L includes at least one thienylene unit as said conjugation-forming unit; and
at least two of the groups existing at the ends of Ls are acceptor groups; 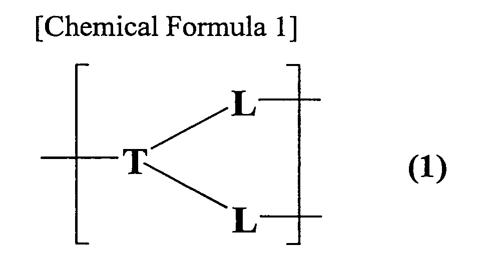 wherein each L represents a divalent organic group which may have a substituent and T represents a trivalent organic group which may have a substituent.

2. The branched compound according to claim 1, wherein a group existing at the end of an L is a group containing a fullerene derivative residue, a group containing a naphthalene imide derivative residue or a group containing a perylene imide derivative residue.

3. The branched compound according to claim 1 or 2, wherein the core part, T(s) and Ls are in conjugation as a whole.

4. The branched compound according to any one of claims 1 to 3, wherein two or more repeating units expressed by the formula (1) are bonded at their Ts to the core part.

5. The branched compound according to any one of claims 1 to 4, wherein an L is a divalent organic group which is expressed by the following formula (2): 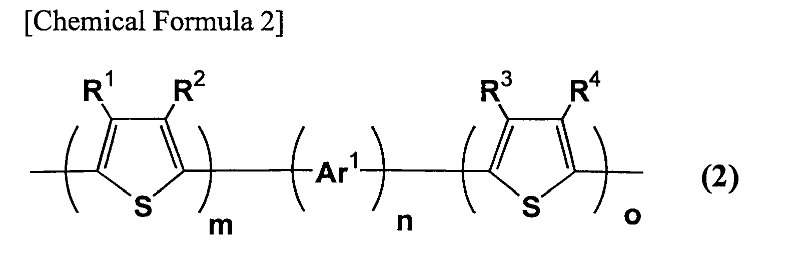 wherein Ar¹ represents a divalent aromatic hydrocarbon group which may have a substituent or a divalent heterocyclic group which may have a substituent; R¹, R², R³ and R⁴ each independently represent a hydrogen atom, an alkyl group, an alkoxy group, an aryl group which may have a substituent, or a monovalent heterocyclic group which may have a substituent, and part or all of hydrogen atoms in each of these groups may be substituted with a fluorine atom; when there are a plurality of R¹s, a plurality of R²s, a plurality of R³s and a plurality of R⁴s, the R¹s may be the same or different from each other, the R²s may be the same or different from each other, the R³s may be the same or different from each other, and the R⁴s may be the same or different from each other; m, n and o each independently represent an integer of 0 to 10, with the proviso that at least one of m and o is an integer of 1 or more, and m+n+o represents an integer of 2 to 16.

6. The branched compound according to any one of claims 1 to 5, wherein the T is any one of trivalent organic groups which are expressed by the following formulae (3) to (7): 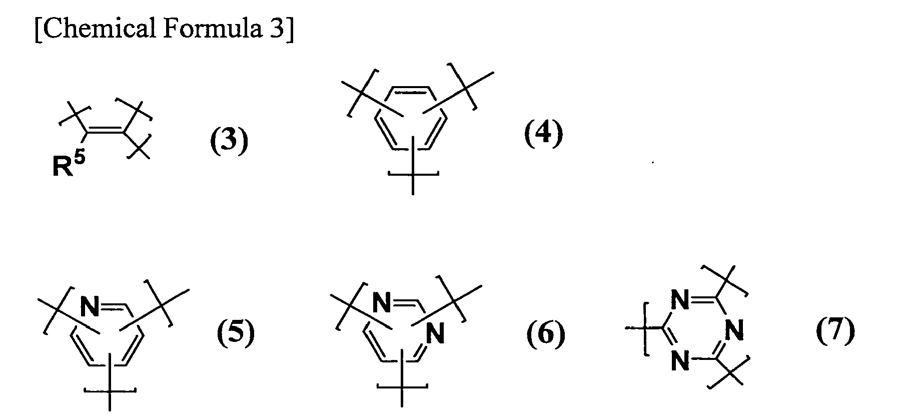 wherein R⁵ represents a hydrogen atom, an alkyl group, an aryl group or a cyano group.

7. The branched compound according to any one of claims 1 to 6, wherein the core part is a divalent organic group which is expressed by the following formula (8): 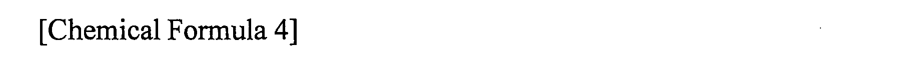 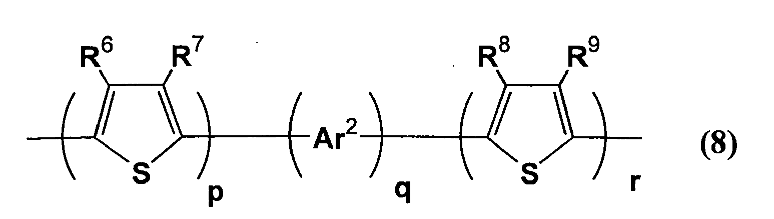 wherein Ar² represents a divalent aromatic hydrocarbon group which may have a substituent or a divalent heterocyclic group which may have a substituent; R⁶, R⁷, R⁸ and R⁹ each independently represent a hydrogen atom, an alkyl group, an alkoxy group, an aryl group which may have a substituent, or a monovalent heterocyclic group which may have a substituent, and part or all of hydrogen atoms in each of these groups may be substituted with a fluorine atom; when there are a plurality of R⁶s, a plurality of R⁷s, a plurality of R⁸s and a plurality of R⁹s, the R⁶s may be the same or different from each other, the R⁷s may be the same or different from each other, the R⁸s may be the same or different from each other, and the R⁹s may be the same or different from each other; p, q and r each independently represent an integer of 0 to 10, with the proviso that p + q + r represents an integer of 2 to 20.

8. The branched compound according to any one of claims 1 to 7, wherein the repeating unit which is expressed by the following formula (1) is a repeating unit which is expressed by the following formula (9): 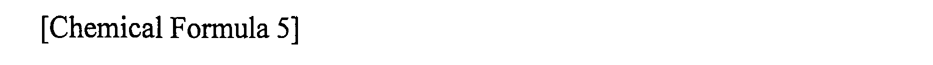 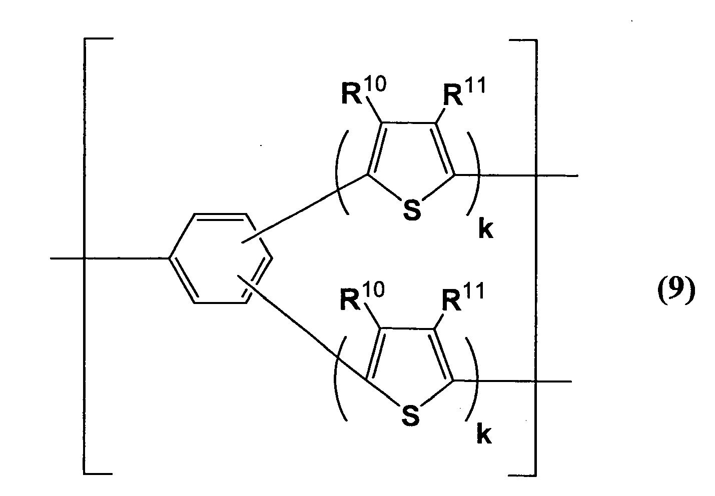 wherein R¹⁰ and R¹¹ each independently represent a hydrogen atom, an alkyl group or an aryl group, and part or all of hydrogen atoms in each of these groups may be substituted with a fluorine atom; R¹⁰ and R¹¹ which plurally exist may be each the same or different; each k represents an integer of 3 to 10; and the ks may be the same or different.

9. An organic thin film comprising the branched compound according to any one of claims 1 to 8.

10. An organic thin film device comprising the organic thin film according to claim 9.

11. An organic thin film transistor comprising the organic thin film according to claim 9.

12. An organic solar cell comprising the organic thin film according to claim 9.

13. A photosensor comprising the organic thin film according to claim 9.
